# EUROPÄISCHE PATENTSCHRIFT

(11) **EP 0 621 037 B1**
(45) Veröffentlichungstag und Bekanntmachung des Hinweises auf die Patenterteilung: **07.07.1999**
(21) Anmeldenummer: 94105958.6
(22) Anmeldetag: 18.04.1994
(51) Int. Cl.: A61K 31/505, C07D 471/04

(54) **Pyrido-pyrimidindione, Verfahren zu ihrer Herstellung und ihre Verwendung als Arzneimittel**
Pyrido-pyrimidinediones, process for their preparation and their use as pharmaceuticals
Composés pyrido-pyrimidinediones, leur préparation et leur utilisation pharmaceutique

(30) Priorität: 23.04.1993 DE 4313317
(43) Veröffentlichungstag der Anmeldung: 26.10.1994
(73) Patentinhaber: HOECHST AKTIENGESELLSCHAFT, 65929 Frankfurt am Main (DE)
(72) Erfinder: Furrer, Harald, Dr., D-65719 Hofheim am Taunus (DE); Seiffge, Dirk, Dr., D-55246 Mainz-Kostheim (DE); Okyayuz-Baklouti, Ismahan, Dr., D-65197 Wiesbaden (DE); Grome, John Joseph, Dr., D-65207 Wiesbaden (DE)

(56) Entgegenhaltungen:
- US-A- 3 186 991

## Beschreibung

Es ist eine Reihe von 5,6,7,8-Tetrahydro-pyrido[4,3-d]-pyrimidinen bekannt, die auch antipyretisch, antiphlogistisch, diuretisch, bakteriostatisch, sedativ und coronardilatorisch wirksam sein können (US 3,186,991).

Es wurde nun gefunden, daß die erfindungsgemäßen Pyrido-pyrimidindione sehr gute antithrombotische Wirkung haben, die Restauration der normalen Funktionen an ischämischen Muskeln fördern sowie günstige Wirkungen auf den Energiestoffwechsel bei neuropathologischen Krankheitsbildern haben. Daher sind die erfindungsgemäßen Pyrido-pyrimidindione geeignet bei der Prophylaxe und/oder Therapie von Durchblutungsstörungen und/oder von neurodegenerativen Erkrankungen eingesetzt zu werden.

Die Erfindung betrifft die Verwendung von mindestens einer Verbindung der Formel I 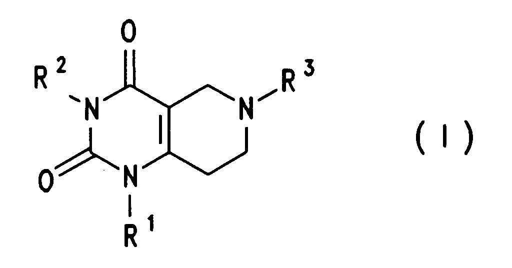 und/oder eines physiologisch verträglichen Salzes der Verbindung der Formel I und/oder einer gegebenenfalls stereoisomeren Form der Verbindung der Formel I, wobei
- R¹: für
a) Wasserstoffatom,
b) (C₁-C₄)-Alkyl,
c) ω-Hydroxy-(C₂-C₄)-alkyl,
d) Benzyl,
e) Benzyl, ein- oder mehrfach am Kern substituiert durch
   1) Halogenatom wie Fluor-, Chlor-, Brom- oder Jodatom,
   2) Nitril oder
   3) Methoxy,
f) (ω-1)-(C₃-C₅)-Alkenyl oder
g) (ω-1)-(C₃-C₅)-Alkenyl,substituiert am (ω-1)-Kohlenstoff durch Methyl, steht,
- R²: für
a) Wasserstoffatom,
b) (C₁-C₆)-Alkyl,
c) Benzyl oder
d) Benzyl, ein- oder mehrfach am Kern substituiert durch
   1) Halogenatom wie Fluor-, Chlor-, Brom- oder Jodatom,
   2) Nitril oder
   3) Methoxy, steht und
- R³: für
a) Wasserstoffatom,
b) (C₁-C₈)-Alkyl,
c) Cyclohexyl-methyl
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
   1.1 Halogenatom wie Fluor-, Chlor-, Brom- oder Jodatom,
   1.2 Nitril,
   1.3 Methoxy oder
   1.4 -CH=CH-COOR⁴, worin R⁴ Wasserstoffatom oder (C₁-C₄)-Alkyl bedeutet,
   und/oder 2) in α-Position des Benzylrestes einfach substituiert durch
   2.1 Methyl,
   2.2 Hydroxymethyl,
   2.3 Carboxyl oder
   2.4 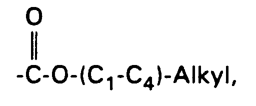
i) -(CH₂)ₙ-COOR⁵, worin
   n eine ganze Zahl von 1 bis 8 darstellt und
   R⁵
      a) Wasserstoffatom oder
      b) (C₁-C₄)-Alkyl bedeutet,
k) -CH₂-CH=CH-COOR⁶, worin R⁶ (C₁-C₄)-Alkyl bedeutet,
l) Pyridylmethyl,
m) 1-Benzyl-imidazol-2-yl-methyl,
n) -CH(COOR⁶)₂, worin R⁶ (C₁-C₄)-Alkyl bedeutet,
o) ω-Morpholin-4-yl-(C₂-C₄)-alkyl,
p) 2-Methylsulfinyl-ethyl oder
q) Thienyl-methyl (Thenyl)
steht, zur Herstellung von Arzneimitteln zur Prophylaxe und Therapie von Durchblutungsstörungen und/oder neurodegenerativen Erkrankungen.

Bevorzugt ist die Verwendung von mindestens einer Verbindung der Formel I und/oder eines physiologisch verträglichen Salzes der Verbindung der Formel I, wobei

- R¹: für
a) Wasserstoffatom,
b) (C₁-C₄)-Alkyl,
c) ω-Hydroxy-(C₂-C₄)-alkyl,
d) Benzyl,
e) Benzyl, ein- oder mehrfach am Kern substituiert durch
   1) Fluor- oder Chloratom,
   2) Nitril oder
   3) Methoxy,
f) (ω-1)-(C₃-C₅)-Alkenyl oder
g) (ω-1)-(C₃-C₅)-Alkenyl, substituiert am (ω-1)-Kohlenstoff durch Methyl, steht,
- R²: für
a) Wasserstoffatom
b) (C₁-C₆)-Alkyl,
c) Benzyl oder
d) Benzyl, ein- oder mehrfach am Kern substituiert durch
   1) Fluor- oder Chloratom,
   2) Nitril oder
   3) Methoxy, steht und
- R³: für
a) Wasserstoffatom,
b) (C₁-C₈)-Alkyl,
c) Cyclohexyl-methyl,
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
   1.1 Fluor- oder Chloratom,
   1.2 Nitril,
   1.3 Methoxy oder
   1.4 -CH=CH-COOR⁴, worin R⁴ Wasserstoffatom oder (C₁-C₄)-Alkyl bedeutet,
   und/oder 2) in α-Position des Benzylrestes einfach substituiert durch
   2.1 Methyl,
   2.2 Hydroxymethyl,
   2.3 Carboxyl,
   2.4 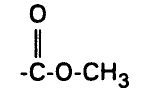 oder
   2.5 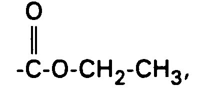
i) -(CH₂)ₙ-COOR⁵, worin
   n eine ganze Zahl von 1 bis 8 darstellt und
   R⁵
      a) Wasserstoffatom oder
      b) (C₁-C₄)-Alkyl bedeutet,
k) -CH₂-CH=CH-COOR⁶, worin R⁶ (C₁-C₄)-Alkyl bedeutet,
l) 2-Pyridylmethyl,
m) 1-Benzyl-imidazol-2-yl-methyl,
n) -CH(COOR⁶)₂, worin R⁶ (C₁-C₄)-Alkyl bedeutet,
o) ω-Morpholin-4-yl-(C₂-C₄)-alkyl,
p) 2-Methylsulfinyl-ethyl oder
q) 2-Thienyl-methyl (2-Thenyl)
steht.

Unter dem Begriff "Benzyl" wird ein Rest der Struktur -CH₂-C₆H₅
verstanden. Der Begriff "Benzyl, substituiert am Kern" bedeutet eine Substitution eines Wasserstoffatoms am C₆H₅-Teil des Benzyls; der Begriff "in α-Position des Benzylrestes einfach substituiert" bedeutet eine Substitution eines Wasserstoffatoms am -CH₂-Teil des Benzyls.

Unter dem Begriff "Alkyl" werden geradkettige und verzweigte Kohlenwasserstoffreste verstanden.

Die Erfindung betrifft ferner Verbindungen der Formel I und physiologisch verträgliche Salze der Verbindung der Formel I und gegebenenfalls stereoisomere Formen der Verbindung der Formel I, wobei
- R¹: für
a) Wasserstoffatom,
b) (C₁-C₄)-Alkyl,
c) ω-Hydroxy-(C₂-C₄)-alkyl,
d) Benzyl,
e) Benzyl, ein- oder mehrfach am Kern substituiert durch
   1) Halogenatom wie Fluor-, Chlor-, Brom- oder Jodatom,
   2) Nitril oder
   3) Methoxy,
f) (ω-1)-(C₃-C₅)-Alkenyl oder
g) (ω-1)-(C₃-C₅)-Alkenyl, substituiert am (ω-1)-Kohlenstoff durch Methyl, steht,
- R²: für
a) Wasserstoffatom,
b) (C₁-C₆)-Alkyl,
c) Benzyl oder
d) Benzyl, ein- oder mehrfach am Kern substituiert durch
   1) Halogenatom wie Fluor-, Chlor-, Brom- oder Jodatom,
   2) Nitril oder
   3) Methoxy, steht und
- R³: für
a) Wasserstoffatom,
b) (C₁-C₈)-Alkyl,
c) Cyclohexyl-methyl,
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
   1.1 Halogenatom wie Fluor-, Chlor-, Brom- oder Jodatom,
   1.2 Nitril,
   1.3 Methoxy oder
   1.4 -CH=CH-COOR⁴, worin R⁴ Wasserstoffatom oder (C₁-C₄)-Alkyl bedeutet,
   und/oder 2) in α-Position des Benzylrestes einfach substituiert durch
   2.1 Methyl,
   2.2 Hydroxymethyl,
   2.3 Carboxyl oder
   2.4 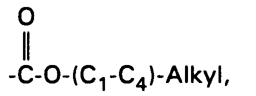
i) -(CH₂)ₙ-COOR⁵, worin
   n eine ganze Zahl von 1 bis 8 darstellt und
   R⁵
      a) Wasserstoffatom oder
      b) (C₁-C₄)-Alkyl bedeutet,
k) -CH₂-CH=CH-COOR⁶, worin R⁶ (C₁-C₄)-Alkyl bedeutet,
l) Pyridylmethyl,
m) 1-Benzyl-imidazol-2-yl-methyl,
n) -CH(COOR⁶)₂, worin R⁶ (C₁-C₄)-Alkyl bedeutet,
o) ω-Morpholin-4-yl-(C₂-C₄)-alkyl,
p) 2-Methylsulfinyl-ethyl oder
q) Thienylmethyl (Thenyl) steht,
wobei die Verbindung der Formel I, worin R¹ und R² Wasserstoffatom sind, ausgenommen ist.

Bevorzugt sind Verbindungen der Formel I und physiologisch verträgliche Salze der Verbindung der Formel I, wobei
- R¹: für
a) Wasserstoffatom,
b) (C₁-C₄)-Alkyl,
c) ω-Hydroxy-(C₂-C₄)-alkyl,
d) Benzyl,
e) Benzyl, ein- oder mehrfach am Kern substituiert durch Fluor- oder Chloratom
f) (ω-1)-(C₃-C₅)-Alkenyl oder
g) (ω-1)-C₃-C₅)-Alkenyl, substituiert am (ω-1)-Kohlenstoff durch Methyl, steht,
- R²: für
a) Wasserstoffatom,
b) (C₁-C₆)-Alkyl,
c) Benzyl oder
d) Benzyl, ein- oder mehrfach am Kern substituiert durch Fluor- oder Chloratom, steht und
- R³: für
a) Wasserstoffatom,
b) (C₁-C₈)-Alkyl,
c) Cyclohexyl-methyl,
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
   1.1 Fluor- oder Chloratom,
   1.2 Nitril,
   1.3 Methoxy oder
   1.4 -CH=CH-COOR⁴, worin R⁴ Wasserstoffatom oder (C₁-C₄)-Alkyl bedeutet,
   und/oder 2) in α-Position des Benzylrestes einfach substituiert durch
   2.1 Methyl,
   2.2 Hydroxymethyl,
   2.3 Carboxyl,
   2.4 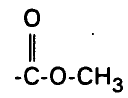 oder
   2.5 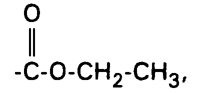
i) -(CH₂)ₙ-COOR⁵, worin
   n eine ganze Zahl von 1 bis 8 darstellt und
   R⁵
      a) Wasserstoffatom oder
      b) (C₁-C₄)-Alkyl bedeutet,
k) -CH₂-CH=CH-COOR⁶, worin R⁶ (C₁-C₄)-Alkyl bedeutet,
l) 2-Pyridylmethyl,
m) 1-Benzyl-imidazol-2-yl-methyl,
n) -CH(COOR⁶)₂, worin R⁶ (C₁-C₄)-Alkyl bedeutet,
o) ω-Morpholin-4-yl-(C₂-C₄)-alkyl,
p) 2-Methylsulfinyl-ethyl oder
q) 2-Thienyl-methyl (2-Thenyl) steht,
wobei die Verbindung der Formel I, worin R¹ und R² Wasserstoffatom sind, ausgenommen ist.

Insbesondere bevorzugt sind Verbindungen der Formel I und physiologisch verträgliche Salze der Formel I, wobei
- R¹: für
a) Wasserstoffatom,
b) (C₁-C₄)-Alkyl,
c) 2-Hydroxyethyl,
d) Benzyl oder
e) 2-Methylallyl, steht,
- R²: für
a) Wasserstoffatom,
b) (C₁-C₆)-Alkyl oder
c) Benzyl steht und
- R³: für
a) Wasserstoffatom,
b) (C₁-C₈)-Alkyl,
c) Cyclohexyl-methyl
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
   1.1 Halogenatom wie Fluor- oder Chloratom,
   1.2 Nitril,
   1.3 Methoxy oder
   1.4 -CH=CH-COOR⁴, worin R⁴ Methyl oder Ethyl bedeutet,
   und/oder 2) in α-Position des Benzylrestes einfach substituiert durch
   2.1 Methyl,
   2.2 Hydroxymethyl,
   2.3 Carboxyl,
   2.4 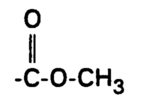 oder
   2.5 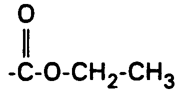 steht,
i) -(CH₂)ₙ-COOR⁵, worin
   n eine ganze Zahl von 1 bis 8 darstellt und
   R⁵
      a) Wasserstoffatom oder
      b) (C₁-C₂)-Alkyl bedeutet,
k) -CH₂-CH=CH-COOR⁶, worin R⁶ (C₁-C₂)-Alkyl bedeutet,
l) 2-Pyridylmethyl,
m) 1-Benzyl-imidazol-2-yl-methyl,
n) -CH(COOR⁶)₂, worin R⁶ (C₁-C₂)-Alkyl bedeutet,
o) ω-Morpholin-4-ylethyl,
p) 2-Methylsulfinyl-ethyl, oder
q) 2-Thienyl-methyl (2-Thenyl) steht,
wobei die Verbindung der Formel I, worin R¹ und R² Wasserstoffatom sind, ausgenommen ist.

Ferner sind Verbindungen der Formel I und physiologisch verträgliche Salze der Verbindung der Formel I bevorzugt, wobei
- R¹: für
a) Wasserstoffatom,
b) Methyl oder
c) Ethyl steht,
- R²: für
a) Wasserstoffatom,
b) (C₁-C₄)-Alkyl oder
c) Benzyl steht,
- R³: für
a) Wasserstoffatom,
b) (C₁-C₈)-Alkyl,
c) Cyclohexyl-methyl
d) ω-Hydroxy-(C₂-C₄)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
   1.1 Fluor- oder Chloratom,
   1.2 Nitril,
   1.3 Methoxy oder
   1.4 -CH=CH-COOR⁴, worin R⁴ Methyl oder Ethyl bedeutet,
   und/oder 2) in α-Position des Benzylrestes einfach substituiert durch
   2.1 Carboxyl,
   2.2 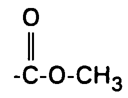 oder
   2.3 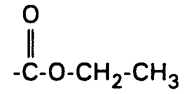 oder
i) 2-Thienyl-methyl (2-Thenyl) steht,
wobei die Verbindung der Formel I, worin R¹ und R² Wasserstoffatom sind, ausgenommen ist.

Die Erfindung betrifft weiterhin Verfahren zur Herstellung der Verbindung der Formel I, wobei eine Ausführungsform darin besteht, daß man
A) eine Verbindung der Formel II 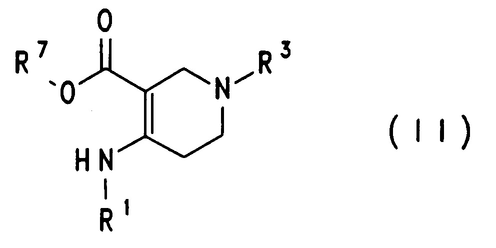 wobei
   - R¹: a) Wasserstoffatom oder
   b) (C₁-C₄)-Alkyl ist,
   - R³: a) (C₁-C₈)-Alkyl,
   b) Cyclohexyl-methyl,
   c) Benzyl, ein- oder mehrfach am Kern mit Halogenatom oder Methoxy substituiert wie in Formel I oder
   d) Benzyl ist und
   - R⁷: einen niederen Alkylrest, vorzugsweise Methyl oder Ethyl,bedeutet, mit einer Verbindung der Formel III

   R² - N = C = Y (III)
   wobei
   - R²: a) (C₁-C₆)-Alkyl,
   b) Benzyl, ein- oder mehrfach am Kern mit Halogenatom oder Methoxy substituiert wie in Formel I, oder
   c) Benzyl ist und
   - Y: Sauerstoffatom bedeutet,
   zu einer Verbindung der Formel I umsetzt 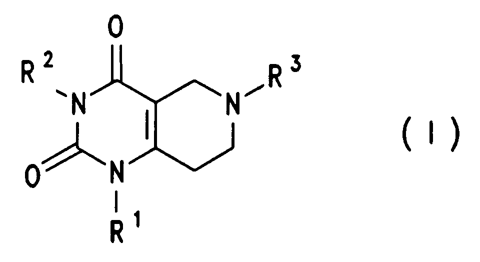 wobei R² die in Formel III sowie R¹ und R³ die in
   Formel II angegebene Bedeutung haben, oder
B) eine Verbindung der Formel II, wobei R¹, R³ und R⁷ die in Formel II angegebene Bedeutung haben, mit einer Verbindung der Formel III, wobei
   - R²: a) (C₁-C₆)-Alkyl,
   b) Benzyl, ein- oder mehrfach am Kern mit Halogenatom oder Methoxy substituiert wie in Formel I, oder
   c) Benzyl ist und
   - Y: Schwefelatom bedeutet,
   zu einer Verbindung der Formel IV umsetzt 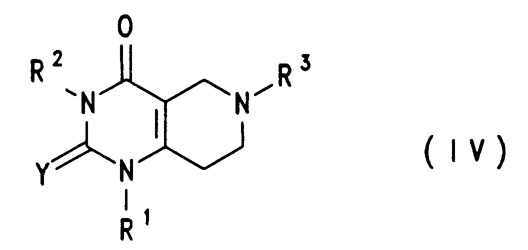 wobei Y Schwefelatom bedeutet und R² die in Formel III sowie R¹ und R³ die in Formel II angegebene Bedeutung haben,
   und die Verbindung der Formel IV zu einer Verbindung der Formel I oxidiert, wobei R² die in Formel III sowie R¹ und R³ die in Formel II angegebene Bedeutung haben, oder
C) eine Verbindung der Formel I, wobei R¹ für Wasserstoffatom, (C₁-C₄)-Alkyl, ω-Hydroxy-(C₂-C₄)-Alkyl und R² für Wasserstoffatom, (C₁-C₆)-Alkyl, Benzyl, Benzyl ein oder mehrfach am Kern substituiert durch Methoxy stehen und R³ ein Benzylrest ist zu einer Verbindung der Formel I umsetzt, wobei R³ für Wasserstoffatom steht und R¹ und R² die hier für die Verbindung der Formel I genannte Bedeutung haben, oder
D) eine Verbindung der Formel I und/oder Salze der Verbindung der Formel I, wobei R¹ und/oder R³ Wasserstoffatom bedeuten und R² die in Formel I angegebene Bedeutung außer Wasserstoff hat, mit einer Verbindung der Formel VII

   R⁸ - X (VII)

   wobei
   - X: für
   a) Halogenatom wie Chlor-, Jod- oder Bromatom,
   b) Sulfonsäureesterrest oder
   c) Phosphorsäureesterrest, und
   - R⁸: die in Formel I für R¹ und R³ angegebene Bedeutung hat, ausgenommen die Bedeutung Wasserstoffatom, wobei man diejenigen Reste R⁸, die eine alkoholische Hydroxylgruppe oder eine Carboxylgruppe tragen, gegebenenfalls in geschützter Form umsetzt und nach Abspaltung der Schutzgruppe in eine Verbindung der Formel I überführt, oder
E) eine Verbindung der Formel I, wobei R¹ und R² die in Formel I genannte Bedeutung haben mit Ausnahme von (ω-1)-(C₃-C₅)-Alkenyl mit Wasserstoff oder Methyl an (ω-1)-Kohlenstoffatom oder Benzyl, ein- oder mehrfach am Kern substituiert durch Nitril, Brom- oder Jodatom und R³ 2-Methylsulfanyl-ethyl ist, mit einem Oxidationsmittel, vorzugsweise einer Persäure, zu einer Verbindung der Formel I umsetzt, wobei R¹ und R² die hier für die Verbindung der Formel I genannte Bedeutung haben und R³ 2-Methylsulfinylethyl ist, oder
F) eine Verbindung der Formel I, wobei R¹ und R² für Wasserstoffatom, Alkyl oder Benzyl gemäß Formel I stehen und R³ Wasserstoffatom ist, mit einer Verbindung der Formel V 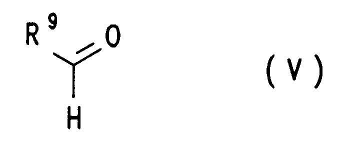 wobei
   - R⁹: a) Wasserstoffatom,
   b) (C₁-C₇)-Alkyl,
   c) Cyclohexyl oder
   d) Phenyl bedeutet,
unter reduktiven Reaktionsbedingungen zu einer Verbindung der Formel I umsetzt.

Bei Verfahrensvariante A geht man beispielsweise so vor, daß man die Verbindungen der Formel II durch Reaktion von Substanzen der Formel VI mit den Aminen R¹-NH₂ in geeigneten Lösungsmitteln, beispielsweise niederen Alkoholen oder deren Gemisch mit Wasser, bei erhöhten Temperaturen, vorzugsweise zwischen 40°C und dem Siedepunkt des jeweiligen Lösungsmittels herstellt, wobei R¹, R³ und R⁷ die für Formel II angegebenen Bedeutungen haben.

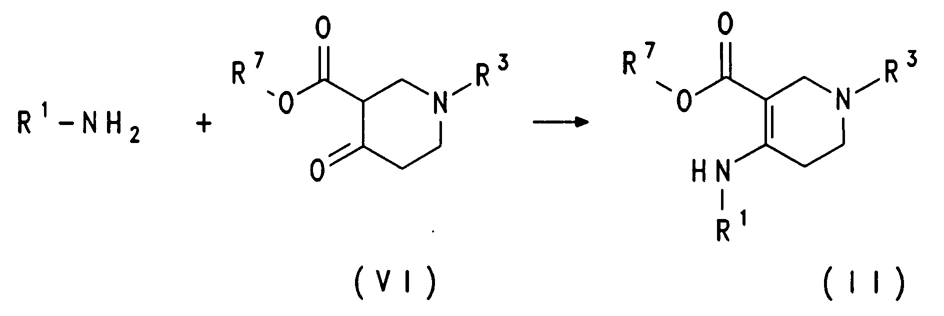

Die Substanzen der Formel VI sind nach bekannten Verfahren zugänglich.

Die Umsetzung der Verbindungen der Formel II mit den Verbindungen der Formel III, in der Y ein Sauerstoffatom bedeutet, zu Verbindungen der Formel I erfolgt beispielsweise in einem inerten Lösungsmittel, vorzugsweise Toluol, in Gegenwart eines tertiären Amins, im allgemeinen von Triethylamin, bei erhöhten Temperaturen bis zum Siedepunkt des jeweiligen Lösungsmittels, gegebenenfalls unter erhöhtem Druck, wobei sich eine Nachbehandlung unter alkalischen Bedingungen, vorzugsweise unter Verwendung von Natrium-ethanolat, in einem geeigneten Lösungsmittel, vorteilhaft Ethanol, unter erhöhter Temperatur, bevorzugt unter Rückflußbedingungen anschließt.

Bei der Verfahrensvariante B geht man wie bei Verfahrensvariante A vor; wobei in der Verbindung der Formel III Y ein Schwefelatom bedeutet. Nachbehandlung unter alkalischen Bedingungen ist nicht nötig. Die Oxidation der Verbindung der Formel IV, wobei Y = Schwefelatom ist, erfolgt beispielsweise in alkalischem Medium mit Wasserstoffperoxid.

Bei der Verfahrensvariante C erfolgt die Debenzylierung unter üblichen Bedingungen, bevorzugt durch katalytische Hydrogenolyse in Gegenwart von Edelmetallkatalysatoren, im allgemeinen Palladium oder Platin, in Lösungsmitteln wie Methanol, Ethanol oder Eisessig bei Normaldruck oder erhöhtem Druck, bevorzugt bei Drucken von 1 bis 5 bar, und bei Temperaturen von Raumtemperatur bis 80°C.

Bei der Verfahrensvariante D erfolgt die Umsetzung in inerten Verteilungs- oder Lösungsmitteln. Als solche kommen vor allem dipolar aprotische Lösungsmittel, beispielsweise Dimethylformamid, Dimethylacetamid, Aceton oder Methylethylketon in Frage, es können aber auch Alkohole, wie etwa Methanol oder Ethanol oder halogenierte Kohlenwasserstoffe, wie Dichlormethan oder Chloroform eingesetzt werden. Diese Umsetzungen werden zweckmäßig in Gegenwart eines basischen Agenses durchgeführt. Hierfür eignen sich im Falle des Ersatzes von R¹ = H durch einen der in Formel I für R¹ genannten Reste besonders Alkali- oder Erdalkalimetall-hydroxide, -carbonate, -alkoholate, -hydride und bei Ersatz von Wasserstoff in der Position von R³ organische Basen, bevorzugt Ethyl-diisopropylamin, Diazabicyclonen oder Diazabicycloundecen. Die Verbindungen der Formel I mit R¹ = H können aber auch unmittelbar in Form ihrer gesondert hergestellten Salze, etwa der Alkali- oder Erdalkalisalze in die Alkylierungsreaktion eingesetzt werden. Bei der Einführung der Alkylreste gemäß den voranstehend beschriebenen Verfahrensweisen arbeitet man im allgemeinen bei einer Reaktionstemperatur zwischen Raumtemperatur und dem Siedepunkt des jeweiligen Lösungsmittels, vorzugsweise zwischen 20°C und 100°C.

Der Schutz derjenigen Alkylierungsmittel R⁸-X, die eine alkoholische Hydroxylgruppe oder einen Carboxylrest enthalten sowie die eventuelle Abspaltung der jeweiligen Schutzgruppe nach erfolgter Alkylierung unter Bildung von Verbindungen der Formel I erfolgt nach bekannten Methoden. Der alkoholische Hydroxylrest in den Verbindungen R⁸-X wird bevorzugt mit 2,3-Dihydropyran oder Isopropenyl-methylether durch Reaktion in saurem Medium im allgemeinen bei Raumtemperatur in einem inerten Lösungsmittel, beispielsweise Dichlormethan, geschützt und die Schutzgruppe nach der Alkylierung bevorzugt durch Hydrolyse oder Methanolyse in saurem Medium zu den Verbindungen der Formel I mit einer alkoholischen Funktion am Substituenten in der Position von R¹ und/oder R³ entfernt.

Der Carbonsäurerest in den Verbindungen R⁸-X wird bevorzugt in Form von Carbonsäure-(C₁-C₄)-alkylestern geschützt, die auf bekannte Weise dargestellt werden. Die Carbonsäure-tert.-butylester werden vorteilhaft durch Reaktion der jeweiligen Carbonsäuren mit Isobutylen in saurem Medium, bevorzugt in Dichlormethan in Gegenwart von Schwefelsäure erhalten. Die Überführung der Carbonsäureester der Formel I in die jeweiligen Carbonsäuren geschieht nach üblichen Methoden, wobei im Falle der Carbonsäure-tert.-butylester deren Reaktion in Dichlormethan mit Chlorwasserstoff bei Temperaturen von 0-5°C bevorzugt ist.

Bei der Verfahrensvariante E erfolgt die Oxidation beispielsweise durch Wasserstoffperoxid in Aceton oder Eisessig, Tetrabutylammoniumperiodat in Chloroform, bevorzugt sind aber organische Persäuren, wie etwa Magnesiummonoperoxy-phthalat in Ethanol. Die Reaktionstemperaturen liegen zwischen 0°C und dem Siedepunkt des jeweiligen Lösungsmittels, vorzugsweise aber bei 0°C bis 60°C.

Bei der Verfahrensvariante F erfolgt die Umsetzung nach der bekannten Methode der reduktiven Alkylierung mit einem Aldehyd R⁹-CHO. Als Reduktionsmittel dienen hauptsächlich katalytisch aktivierter Wasserstoff sowie Ameisensäure und ihre Derivate (Leuckart-Wallach-Reaktion).

Die Erfindung betrifft auch Arzneimittel, gekennzeichnet durch einen wirksamen Gehalt an mindestens einer Verbindung der Formel I oder eines ihrer physiologisch verträglichen Salze neben pharmazeutisch geeigneten und physiologisch verträglichen Trägerstoffen, Zusatzstoffen und/oder anderen Wirk- und Hilfsstoffen.

Die erfindungsgemäßen Arzneimittel können oral, topisch, rektal, intravenös oder gegebenenfalls auch parenteral appliziert werden.

Die erfindungsgemäßen Arzneimittel eignen sich vorzugsweise zur Prophylaxe und/oder Therapie von Störungen der Durchblutung und des Stoffwechsels, insbesondere von Störungen im Bereich der Mikrozirkulation sowie von daraus resultierenden Erkrankungen.

Die aus Durchblutungsstörungen, insbesondere aus Störungen im Bereich der Mikrozirkulation resultierenden Erkrankungen sind hauptsächlich ischämische Skelett- und/oder Herzmuskelerkrankungen, insbesondere Claudicatio intermittens, Ulcus cruris sowie degenerative und/oder entzündliche Muskelerkrankungen verschiedener Genese mit oder ohne Muskelatrophie, Vasculitiden mit thrombotischen Ereignissen, arterielle und venöse Blutgerinnsel, z.B. Thrombosen, Schock oder Infarkt.

Wegen der durchblutungsfördernden Wirkung der erfindungsgemäßen Verbindungen und Arzneimittel, insbesondere im Mikrobereich, sind die Verbindungen und Arzneimittel auch wirksam bei Arteriosklerose, bei der Operationsnachbehandlung zur Verhinderung postoperativer Thrombosen, zur Nachbehandlung von Krebs zwecks Verhinderung bzw. Verminderung der Metastasierung, bei der Behandlung von Patienten, die an Herz-Lungen-Maschinen oder die Nierendialyse angeschlossen sind und schließlich auch von Patienten nach transienten ischämischen Attacken (TIA), Schlaganfall oder Herzinfarkt sowie zur Wundheilung nach Traumen und exogenen Noxen.

Die erfindungsgemäßen Arzneimittel eignen sich auch zur Prophylaxe und/oder Therapie von neurodegenerativen Erkrankungen bei Mensch und Tier. Eine große Zahl neuropathologischer Situationen sind durch eine Degeneration sowie den Verlust von Neuronen charakterisiert. Dies gilt insbesondere für neurodegenerative Krankheitsbilder, wie Schlaganfall, temporäre Hirnischämie (TIA), zerebraler Infarkt mit nur teilweise reversiblen Symptomen (PRIND), Gehirnlähmung, zerebrale Hypoglycämie, ischämische Ereignisse während Herzstillstand oder chirurgischer Eingriffe im Herz-Lungen-Bereich, anoxische Zustände, zum Beispiel nach Ertrinken, Intoxikation oder Rückenmarksverletzungen, perinatale Asphyxie, altersbedingte neurodegenerative Veränderungen, Alzheimer-Demenz (SDAT), Schmerz, Übersekretion von Wachstumshormon und Luteinisierungshormon, Schizophrenie, Epilepsie, Huntington-Chorea sowie andere chronische neurodegenerative Erkrankungen.

Die Erfindung betrifft auch ein Verfahren zur Herstellung eines erfindungsgemäßen Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykole und Lösungsmittel, wie etwa steriles Wasser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I, bei Mensch und Tier Tagesdosen von etwa 50 bis 3000 mg Wirkstoff, vorzugsweise etwa 150 bis 1000 mg, bei oraler Verabreichung und von etwa 50 bis 1000 mg, bevorzugt etwa 100 bis 300 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen. Schließlich können die Verbindungen der Formel I und/oder gegebenenfalls ihre physiologisch verträglichen Salze bei der Herstellung der vorgenannten galenischen Darreichungsformen auch zusammen mit anderen geeigneten Wirkstoffen, beispielsweise durchblutungsfördernden Substanzen, Plättchenaggregationshemmer, Thrombozytenaggregationshemmern und Calciumantagonisten, Antithrombotika, Antihyperlipidämika, Neuroprotektiva, Analgetika, Sedativa, Antidepressiva, Antiinflammatorika, antianginösen Mitteln, Cardiotonika, Antiarrhytmika, Diuretika, Antihypertensiva einschließlich β-Rezeptoren- und Calcium-Blockern, Plasmaexpandern und anderen Vasotherapeutika, formuliert werden.

### Beispiele

Die Struktur aller nachstehend beschriebenen Verbindungen wurde durch ¹H-NMR- und IR-Spektren, teilweise auch durch Massenspektren, sowie durch Elementaranalysen abgesichert.

### Abkürzungen:

- a: analog
- B: Beispiel
- DMF: Dimethylformamid
- Fp: Schmelzpunkt resp. Zersetzungsbeginn (Z)
- GC: Gaschromatographie
- Herstg: Herstellung
- iv: intravenös
- ip: intraperitoneal
- konz.: konzentriert
- po: per os
- PVD: peripheral vascular disease
- s: siehe
- sc: subkutan
- Vbg: Verbindung

### Beispiel 1

### 3-Methyl-5,6,7,8-tetrahydro-1H-pyrido[4,3-d]pyrimidin-2,4-dion-hydrochlorid

### a) 4-Amino-1-benzyl-1,2,5,6-tetrahydro-pyridin-3-carbonsäuremethylester

855 g 1-Benzyl-4-oxo-piperidin-3-carbonsäure-methylester-hydrochlorid, 176 g Natriumcarbonat und 450 ml 25%ige wäßrige Ammoniaklösung in 6,5 l Ethanol wurden unter Rühren 5 h bei 70°C Innentemperatur erhitzt. Nach dem Einengen unter vermindertem Druck arbeitete man mit Dichlormethan und Wasser extraktiv auf. Die vereinigten Dichlormethanphasen wurden über Natriumsulfat getrocknet und unter vermindertem Druck eingeengt. Man erhielt 788 g öligen 4-Amino-1-benzyl-1,2,5,6-tetrahydropyridin-3-carbonsäuremethylester als Rohprodukt, das ohne weitere Reinigung in Beispiel 1b) eingesetzt wurde.

### b) 6-Benzyl-3-methyl-2-thioxo-2,3,5,6,7,8-hexahydro-1H-pyrido[4,3-d]pyrimidin-4-on

Man löste 788 g 4-Amino-1-benzyl-1,2,5,6-tetrahydro-pyridin-3-carbonsäuremethylester aus a) in 2,5 l Toluol und versetzte unter Rühren mit 447 g Methylisothiocyanat und 625 ml Triethylamin. Nach 24 h Rühren bei 100°C Innentemperatur ließ man auf 20°C abkühlen, saugte den Niederschlag ab und wusch mit Toluol. Nach Trocknen erhielt man 420 g rohes 6-Benzyl-3-methyl-2-thioxo-2,3,5,6,7,8-hexahydro-1H-pyrido[4,3-d]pyrimidin-4-on, das in c) weiter umgesetzt wurde.
Zersetzungspunkt: 210°C

| C₁₅H₁₇N₃OS (MG = 287.39) | | | | |
|---|---|---|---|---|
| Ber. | C 62,69 % | H 5,96 % | N 14,62 % | S 11,16 % |
| Gef. | C 62,66 % | H 6,03 % | N 14,62 % | S 11,26 % |

### c) 6-Benzyl-3-methyl-5,6,7,8-tetrahydro-1H-pyrido[4,3-d]pyrimidin-2,4-dion

Zu der Suspension von 210 g 6-Benzyl-3-methyl-2-thioxo-2,3,5,6,7,8-hexahydro-1H-pyrido[4,3-d]pyrimidin-4-on aus b) in der Lösung von 78 g NaOH in 4 l Wasser tropfte man bei 0°C unter Rühren innerhalb von 5 1/2 h 3,2 l 30%iges Wasserstoffperoxid. Nach 1/2 Stunde Nachrühren bei 0°C wurde mit konz. Salzsäure der pH von 9,7 auf pH = 7,4 eingestellt, der Niederschlag abfiltriert, mit Wasser gewaschen und getrocknet. Der obige Ansatz wurde wiederholt, und die Niederschläge wurden vereinigt (396 g). Diese wurden zur Reinigung in 1 l Wasser suspendiert und mit 33%iger Natronlauge zur Lösung gebracht (pH 13,5). Man versetzte unter Rühren mit Aktivkohle, filtrierte und stellte das Filtrat auf pH = 10. Der entstandene Niederschlag wurde abgesaugt, mit Wasser gewaschen und unter vermindertem Druck getrocknet. Man erhielt 192 g 6-Benzyl-3-methyl-5,6,7,8-tetrahydro-1H-pyrido[4,3-d]pyrimidin-2,4-dion, das unter d) hydrogenolytisch debenzyliert wurde.

### d) 3-Methyl-5,6,7,8-tetrahydro-1H-pyrido[4,3-d]pyrimidin-2,4-dion

Eine Lösung von 96 g 6-Benzyl-3-methyl-5,6,7,8-tetrahydro-1H-pyrido[4,3-d]pyrimidin-2,4-dion aus c) in 1,5 l Eisessig wurde an 14,6 g 10 % Palladium/Aktivkohle bei 25°C und 3,5 bar während 6 h hydrogenolytisch debenzyliert. Nach Abfiltrieren des Katalysators engte man unter vermindertem Druck ein. Der obige Ansatz wurde wiederholt. Man vereinigte beide Rohprodukte, löste in Wasser und fällte unter Rühren durch Zugabe von 33%iger Natronlauge bis zum pH = 6,9 aus. Der Niederschlag wurde mit Wasser gewaschen und bei 60°C unter vermindertem Druck getrocknet. Man erhielt 115,3 g 3-Methyl-5,6,7,8-tetrahydro-1H-pyrido[4,3-d]pyrimidin-2,4-dion.

### e) 3-Methyl-5,6,7,8-tetrahydro-1H-pyrido[4,3-d]pyrimidin-2,4-dion-hydrochlorid

7,5 g 3-Methyl-5,6,7,8-tetrahydro-1H-pyrido[4,3-d]pyrimidin-2,4-dion wurden in Ethanol gelöst und mit einem 50 %igen Überschuß an etherischer HCl als Hydrochlorid ausgefällt. Der Niederschlag wurde aus Methanol/Diethylether umkristallisiert.
Ausbeute: 7 g
Zersetzungspunkt: 304°C

| C₈H₁₂ClN₃O₂ (MG = 217,7) | | | | |
|---|---|---|---|---|
| Ber. | C 44,15 % | H 5,56 % | Cl 16,29 % | N 19,31 % |
| Gef. | C 44,21 % | H 5,68 % | Cl 16,11 % | N 19,21 % |

### Beispiel 2

### 6-Benzyl-3-methyl-5,6,7,8-tetrahydro-1H-pyrido[4,3-d]pyrimidin-2,4-dion-hydrochlorid

### a) 6-Benzyl-3-methyl-5,6,7,8-tetrahydro-1H-pyrido[4,3-d]pyrimidin-2,4-dion

In einem 5 l Stahlautoklaven werden 530 g 4-Amino-1-benzyl-1,2,5,6-tetrahydropyridin-3-carbonsäuremethylester mit 510 ml Methylisocyanat und 48 g Triethylamin in 2 l trockenem Toluol unter Stickstoff 8 h bei 100°C geschüttelt. Nach dem Abkühlen wurde das Reaktionsgemisch unter vermindertem Druck eingeengt. Den Rückstand löste man in 500 ml Ethanol und erhitzte unter Rühren nach Zugabe von 154 g Natriumethanolat in 1,5 l Ethanol 2 h bei Rückflußtemperatur. Nach dem Einengen dieses Gemisches unter vermindertem Druck arbeitete man den Rückstand zwischen Dichlormethan und Wasser extraktiv auf und stellte die vereinigten wäßrigen Phasen (pH = 12,5) unter Rühren mit konz. Salzsäure auf pH = 10. Der Niederschlag aus 6-Benzyl-3-methyl-5,6,7,8-tetrahydro-1H-pyrido[4,3-d]pyrimidin-2,4-dion wurde abfiltriert, mit Wasser neutral gewaschen und bei 50°C unter vermindertem Druck getrocknet.
Ausbeute: 179,6 g

### b) 6-Benzyl-3-methyl-5,6,7,8-tetrahydro-1H-pyrido[4,3-d]pyrimidin-2,4-dion-hydrochlorid

14,3 g 6-Benzyl-3-methyl-5,6,7,8-tetrahydro-1H-pyrido[4,3-d]pyrimidin-2,4-dion wurden in 300 ml Dichlormethan gelöst und mit etherischer Salzsäure 6-Benzyl-3-methyl-5,6,7,8-tetrahydro-1H-pyrido[4,3-d]pyrimidin-2,4-dion-hydrochlorid unter Eiskühlung ausgefällt. Nach Reinigung durch Umkristallisation aus Isopropanol erhielt man:
Ausbeute: 12,4 g
Zersetzungspunkt: 298°C

| C₁₅H₁₈ClN₃O₂ (MG = 307,78) | | | | |
|---|---|---|---|---|
| Ber. | C 58,54 % | H 5,90 % | Cl 11,52 % | N 13,65 % |
| Gef. | C 58,34 % | H 5,84 % | Cl 11,33 % | N 13,61 % |

### Beispiel 3

### 6-Benzyl-1,3-dimethyl-5,6,7,8-tetrahydro-1H-pyrido[4,3-d]pyrimidin-2,4-dion-hydrochlorid

Zu 40,7 g 6-Benzyl-3-methyl-5,6,7,8-tetrahydro-1H-pyrido[4,3-d]pyrimidin-2,4-dion und 20,7 g Kaliumcarbonat in 230 ml Dimethylformamid tropfte man bei 40°C unter Rühren innerhalb von 10 Minuten 9,6 ml Methyljodid. Nach 8-stündigem Erhitzen auf 60°C engte man unter vermindertem Druck ein, nahm den Rückstand mit Wasser auf, stellte auf pH = 8 und arbeitete mit Dichlormethan und Wasser extraktiv auf. Nach Einengen der gesammelten Dichlormethanphasen unter vermindertem Druck wurde der Rückstand an Kieselgel mit Dichlormethan/Ethanol (Volumenverhältnis 95:5) chromatographisch gereinigt. Man erhielt 16,5 g 6-Benzyl-1,3-dimethyl-5,6,7,8-tetrahydro-1H-pyrido[4,3-d]pyrimidin-2,4-dion, aus dem in Diethylether mit etherischer Salzsäure das 6-Benzyl-1,3-dimethyl-5,6,7,8-tetrahydro-1H-pyrido[4,3-d]pyrimidin-2,4-dion-hydrochlorid als Niederschlag erhalten wurde. Nach dessen Rekristallisation aus Methanol/Diethylether und Entfernen von Restmethanol durch Versetzen des Niederschlags mit Wasser, Einengen unter vermindertem Druck und Trocknen des Rückstandes im Hochvakuum erhielt man:
Ausbeute: 15,1 g
Schmp. 141-143°C

| C₁₆H₂₀ClN₃O₂ mit 1,2 H₂O (MG = 343,43) | | | | |
|---|---|---|---|---|
| Ber. | C 55,96 % | H 6,57 % | Cl 10,32 % | N 12,24 % |
| Gef. | C 56,09 % | H 6,39 % | Cl 10,15 % | N 12,17 % |

### Beispiel 4

### 3,6-Dibenzyl-5,6,7,8-tetrahydro-1H-pyrido[4,3-d]pyrimidin-2,4-dion-hydrochlorid

Die Mischung aus 165 g 4-Amino-1-benzyl-1,2,5,6-tetrahydro-pyridin-3-carbonsäuremethylester, 68 g Triethylamin und 100 g Benzylisocyanat in 700 ml Toluol wurde 8 h unter Rühren auf Rückflußtemperatur erhitzt. Nach dem Abkühlen wurde das Reaktionsgemisch unter vermindertem Druck eingeengt. Den Rückstand löste man in 300 ml Ethanol und erhitzte unter Rühren nach Zugabe von 58 g Natrium-ethanolat in 400 ml Ethanol 1,5 h bei 80°C. Nach dem Einengen dieses Gemisches unter vermindertem Druck arbeitete man den Rückstand zwischen Dichlormethan und Wasser extraktiv auf und engte die vereinigten und getrockneten Dichlormethanextrakte unter vermindertem Druck ein. Die wäßrigen Phasen stellte man gemeinsam mit konz. Salzsäure auf pH = 7. Den entstandenen Niederschlag vereinigte man nach Trocknen mit den eingeengten Dichlormethanextrakten und reinigte durch Umkristallisation aus Ethanol. Man erhielt 81 g 3,6-Dibenzyl-5,6,7,8-tetrahydro-1H-pyrido-[4,3-d]pyrimidin-2,4-dion. 10 g dieser Base wurden in Dichlormethan gelöst und durch Zugabe von etherischer Salzsäure das Hydrochlorid ausgefällt. Nach Umkristallisation aus Ethanol/Wasser (Volumenverhältnis 1:1) erhielt man 10,1 g 3,6-Dibenzyl-5,6,7,8-tetrahydro-1H-pyrido[4,3-d]pyrimidin-2,4-dion-hydrochlorid.
Zersetzung 300°C

| C₂₁H₂₂ClN₃O₂ (MG = 383,88) | | | | |
|---|---|---|---|---|
| Ber. | C 65,71 % | H 5,78 % | Cl 9,24 % | N 10,95 % |
| Gef. | C 65,54 % | H 5,82 % | Cl 9,06 % | N 10,87 % |

### Beispiel 5

### (3-Methyl-2,4-dioxo-2,3,4,5,7,8-hexahydro-1H-pyrido[4,3-d]pyrimidin-6-yl)-phenyl-essigsäure-ethylester-hydrochlorid

Man rührte 11 g 3-Methyl-5,6,7,8-tetrahydro-1H-pyrido[4,3-d]pyrimidin-2,4-dion, 12,7 g Chlor-phenyl-essigsäure-ethylester und 8,3 g Ethyl-diisopropyl-amin in 250 ml Dimethylformamid 48 h bei Raumtemperatur. Nach Einengen im Vakuum arbeitete man mit Dichlormethan und Wasser extraktiv auf. Die Dichlormethanphasen führten nach Trocknen und Entfernen des Lösungsmittels zu 26,6 g öligem Rückstand, aus dem nach Lösen in 50 ml Dichlormethan und Ausfällen mit etherischer Salzsäure 20,9 g (3-Methyl-2,4-dioxo-2,3,4,5,7,8-hexahydro-1H-pyrido[4,3-d]pyrimidin-6-yl)-phenyl-essigsäure-ethylester-hydrochlorid erhalten wurden. Die Umkristallisation erfolgte aus Aceton/Wasser.
Zersetzung: 166°C

| C₁₈H₂₂ClN₃O₄ mit 0,5 H₂O (MG = 388,86) | | | | |
|---|---|---|---|---|
| Ber. | C 55,60 % | H 5,96 % | Cl 9,12 % | N 10,81 % |
| Gef. | C 55,77 % | H 5,92 % | Cl 9,02 % | N 10,81 % |

### Beispiel 6

### (3-Methyl-2,4-dioxo-2,3,4,5,7,8-hexahydro-1H-pyrido[4,3-d]pyrimidin-6-yl)-phenyl-essigsäure-hydrochlorid

Eine Lösung von 15,5 g (3-Methyl-2,4-dioxo-2,3,4,5,7,8-hexahydro-1H-pyrido[4,3-d]pyrimidin-6-yl)-phenyl-essigsäure-ethylester in 150 ml Ethanol/Wasser (Volumenverhältnis 1:1) wurde mit 3,6 g Natriumhydroxyd versetzt und 3 h bei Rückflußtemperatur erhitzt. Nach dem Abkühlen neutralisierte man mit 1 n Salzsäure und engte unter vermindertem Druck zur Trockne ein. Den Rückstand kochte man mehrfach mit Ethanol aus und engte die vereinigten Extrakte unter vermindertem Druck ein. Man erhielt 11,8 g, die in 200 ml Methanol gelöst wurden. Mittels ethanolischer Salzsäure wurde (3-Methyl-2,4-dioxo-2,3,4,5,7,8-hexahydro-1H-pyrido[4,3-d]pyrimidin-6-yl)-phenyl-essigsäure-hydrochlorid als weißer Niederschlag gefällt, der aus Aceton/Wasser umkristallisiert wurde.
Ausbeute: 8,3 g
Zersetzungspunkt 237°C

| C₁₆H₁₈ClN₃O₄ mit 1,5 H₂O (MG = 378,82) | | | | |
|---|---|---|---|---|
| Ber. | C 50,73 % | H 5,59 % | Cl 9,36 % | N 11,09 % |
| Gef. | C 50,81 % | H 5,62 % | Cl 9,19 % | N 11,09 % |

### Beispiel 7

### (3-Benzyl-2,4-dioxo-2,3,4,5,7,8-hexahydro-1H-pyrido[4,3-d]pyrimidin-6-yl)-(2-chlorphenyl)-essigsäure-ethylester-hydrochlorid

### a) 3-Benzyl-5,6,7,8-tetrahydro-1H-pyrido[4,3-d]pyrimidin-2,4-dion

Eine Lösung von 70 g 3,6-Dibenzyl-5,6,7,8-tetrahydro-1H-pyrido[4,3-d]pyrimidin-2,4-dion in 1,5 l Eisessig wurde an 10 g 10 % Palladium/Aktivkohle bei 70°C und 3,5 bar Wasserstoffdruck hydrogenolytisch in 6-Position debenzyliert. Nach Abfiltrieren des Katalysators engte man unter vermindertem Druck ein, nahm das verbleibende Öl mit 500 ml Wasser auf und stellte mit konz. Sodalösung unter Rühren auf pH = 8. Man erhielt 46 g weißen Niederschlag aus 3-Benzyl-5,6,7,8-tetrahydro-1H-pyrido[4,3-d]pyrimidin-2,4-dion.
Schmelzpunkt: 224-226°C (aus Isopropanol)

| C₁₄H₁₅N₃O₂ (MG = 257,30) | | | |
|---|---|---|---|
| Ber. | C 65,35 % | H 5,88 % | N 16,33 % |
| Gef. | C 65,46 % | H 5,89 % | N 16,26 % |

### b) 3-Benzyl-5,6,7,8-tetrahydro-1H-pyrido[4,3-d]pyrimidin-2,4-dion-hydrochlorid

9,4 g 3-Benzyl-5,6,7,8-tetrahydro-1H-pyrido[4,3-d]pyrimidin-2,4-dion wurden in 250 ml Wasser suspendiert, mit der äquivalenten Menge 1N HCl versetzt sowie die entstandene Lösung filtriert und im Vakuum eingeengt. Aus dem Rückstand erhielt man nach Reinigung durch Umkristallisation aus Isopropanol 9,9 g 3-Benzyl-5,6,7,8-tetrahydro-1H-pyrido[4,3-d]pyrimidin-2,4-dion-hydrochlorid.
Schmelzpunkt > 300°C

| C₁₄H₁₆ClN₃O₂ (MG = 293,76) | | | | |
|---|---|---|---|---|
| Ber. | C 57,24 % | H 5,49 % | Cl 12,07 % | N 14,30 % |
| Gef. | C 57,09 % | H 5,56 % | Cl 12,07 % | N 14,27 % |

### c) (3-Benzyl-2,4-dioxo-2,3,4,5,7,8-hexahydro-1H-pyrido[4,3-d]pyrimidin-6-yl)-(2-chlorphenyl)-essigsäure-ethylester-hydrochlorid

Man rührte das Gemisch von 77,2 g 3-Benzyl-5,6,7,8-tetrahydro-1H-pyrido[4,3-d]pyrimidin-2,4-dion, 63 g Ethyl-diisopropyl-amin und 126 g Chlor-(2-chlorphenyl)-essigsäure-ethylester (89%ig gemäß GC) in 1700 ml Dimethylformamid 48 h bei Raumtemperatur. Man engte unter vermindertem Druck ein und arbeitete den Rückstand mit Dichlormethan und Wasser extraktiv auf. Die Dichlormethanphasen wurden vereinigt, getrocknet und eingeengt. Den Rückstand verdünnte man mit 50 ml Dichlormethan versetzte bei Raumtemperatur unter Rühren tropfenweise mit 1,3 Äquivalenten etherischer Salzsäure und langsam mit 700 ml tert.-Butyl-methylether. Man erhielt zunächst ein Öl, das sich allmählich zu einer weißen Masse verfestigte. Dieses (3-Benzyl-2,4-dioxo-2,3,4,5,7,8-hexahydro-1H-pyrido[4,3-d]pyrimidin-6-yl)-(2-chlorphenyl)-essigsäure-ethylester-hydrochlorid wurde durch Umkristallisation aus Dichlormethan/Diethylether gereinigt.
Ausbeute: 112 g
Schmelzpunkt 165-167°C

| C₂₄H₁₅Cl₂N₃O₄ (MG = 490,39) | | | |
|---|---|---|---|
| Ber. | C 58,78 % | H 5,14 % | N 8,57 % |
| Gef. | C 58,60 % | H 5,19 % | N 8,47 % |

### Beispiel 8

### 6-(2-Hydroxyethyl)-3-methyl-5,6,7,8-tetrahydro-1H-pyrido[4,3-d]pyrimidin-2,4-dion

### a) 3-Methyl-6-[2(tetrahydro-pyran-2-yloxy)-ethyl]-5,6,7,8-tetrahydro-1H-pyrido[4,3-d]pyrimidin-2,4-dion

Man versetzte 54,5 g 3-Methyl-5,6,7,8-tetrahydro-1H-pyrido[4,3-d]pyrimidin-2,4-dion in 1,5 l Dimethylformamid unter Rühren mit 40 g Ethyl-diisopropyl-amin sowie 52,5 g 2-(2-Chlorethoxy)-tetrahydro-pyran (96%ig gemäß GC) und erhitzte 30 h bei 70°C. Man engte im Vakuum ein und erhielt nach extraktiver Aufarbeitung des Rückstandes mit Dichlormethan und Wasser sowie Trocknen und Einengen der gesammelten Dichlormethanphasen 25 g 3-Methyl-6-[2-(tetrahydro-pyran-2-yloxy)-ethyl]-5,6,7,8-tetrahydro-1H-pyrido[4,3-d]pyrimidin-2,4-dion, das durch Kugelrohrdestillation bei 80°C Badtemperatur und 0,1 mbar sowie durch Säulenchromatographie an Aluminiumoxid (Aktivitätsstufe III) und Elution mit Dichlormethan/Ethanol (Volumenverhältnis 95:5) gereinigt wurde.

### b) 6-(2-Hydroxyethyl)-3-methyl-5,6,7,8-tetrahydro-1H-pyrido[4,3-d]pyrimidin-2,4-dion

Man löste 22 g 3-Methyl-6-[2-(tetrahydro-pyran-2-yloxy)-ethyl]-5,6,7,8-tetrahydro-1H-pyrido[4,3-d]pyrimidin-2,4-dion in 100 ml Methanol und stellte unter Rühren bei Raumtemperatur mit konz. Salzsäure auf pH = 1. Nach weiteren 7 h wurde unter vermindertem Druck zur Trockne eingeengt, der Rückstand mit Wasser aufgenommen und die Lösung auf pH = 8 gestellt. Man engte unter vermindertem Druck ein und extrahierte den Rückstand erschöpfend mit kaltem Ethanol. Man dampfte den ethanolischen Extrakt unter vermindertem Druck ein und reinigte den Rückstand aus 6-(2-Hydroxyethyl)-3-methyl-5,6,7,8-tetrahydro-1H-pyrido[4,3-d]pyrimidin-2,4-dion durch Umkristallisation aus Isopropanol sowie Ethanol.
Ausbeute: 5 g
Schmelzpunkt 156°C

| C₁₀H₁₅N₃O₃ mit 0,5 H₂O (MG = 234,25) | | | |
|---|---|---|---|
| Ber. | C 51,27 % | H 6,88 % | N 17,94 % |
| Gef. | C 51,08 % | H 6,62 % | N 17,92 % |

### Beispiel 9

### 6-(2-Hydroxy-1-phenyl-ethyl)-3-methyl-5,6,7,8-tetrahydro-1H-pyrido[4,3-d]pyrimidin-2,4-dion-hydrochlorid

Man rührte 17,2 g 3-Methyl-5,6,7,8-tetrahydro-1H-pyrido[4,3-d]pyrimidin-2,4-dion, 23 g 2-(2-Chlor-2-phenyl-ethoxy)-tetrahydro-pyran (aus 2-Chlor-2-phenyl-ethanol und 3,4-Dihydro-2H-pyran) und 17,5 g Ethyl-diisopropyl-amin 45 h bei 80°C und 24 h bei 100°C. Nach Einengen des Ansatzes unter vermindertem Druck wurde der Rückstand mit Dichlormethan und Wasser extraktiv aufgearbeitet. Die vereinigten Dichlormethanphasen engte man nach Trocknen ein und reinigte den Rückstand durch Chromatographie in einer Mitteldrucksäule an Aluminiumoxid (Aktivitätsstufe III) mit Dichlormethan/Ethanol (Volumenverhältnis 97:3) als Laufmittel. Man erhielt 8,5 g 3-Methyl-6-[2-(tetrahydropyran-2-yloxy)-1-phenyl-ethyl]-3-methyl-5,6,7,8-tetrahydro-1H-pyrido[4,3-d]pyrimidin-2,4-dion, aus denen in einem Gemisch aus 15 ml 2n Salzsäure und 25 ml Glykoldimethylether durch 2-stündiges Erhitzen auf 88°C, gefolgt vom Einstellen des pH auf 7,4, Einengen unter vermindertem Druck und extraktiver Aufarbeitung des erhaltenen Rückstandes zwischen Dichlormethan und Wasser nach Einengen der getrockneten Dichlormethanphasen 6-(2-Hydroxy-1-phenyl-ethyl)-3-methyl-5,6,7,8-tetrahydro-1H-pyrido-[4,3-d]pyrimidin-2,4-dion erhalten wurde. Dieses reinigte man durch Säulenchromatographie an Kieselgel mit Dichlormethan/Ethanol (Volumenverhältnis 95:5) als Laufmittel. Aus den resultierenden 7,6 g erhielt man in Dichlormethan mit etherischer Salzsäure 6-(2-Hydroxy-1-phenyl-ethyl)-3-methyl-5,6,7,8-tetrahydro-1H-pyrido-[4,3-d]pyrimidin-2,4-dion-hydrochlorid, das durch Umkristallisation aus Isopropanol gereinigt wurde.
Ausbeute: 4,5 g
Zersetzung 150°C

| C₁₆H₂₀ClN₃O₃ mit 0,3 H₂O (MG = 343,21) | | | |
|---|---|---|---|
| Ber. | C 55,99 % | H 6,05 % | N 12,24 % |
| Gef. | C 55,97 % | H 5,84 % | N 12,15 % |

### Beispiel 10

### 3-Methyl-6-(2-methylsulfinyl-ethyl)-5,6,7,8-tetrahydro-1H-pyrido[4,3-d]pyrimidin-2,4-dion

### a) 3-Methyl-6-(2-methylsulfanyl-ethyl)-5,6,7,8-tetrahydro-1H-pyrido[4,3-d]pyrimidin-2,4-dion

Man rührte 36,3 g 3-Methyl-5,6,7,8-tetrahydro-1H-pyrido[4,3-d]pyrimidin-2,4-dion, 23 g 1-Chlor-2-methylsulfanyl-ethan (97%ig), 17,4 g Lithiumbromid und 27 g Ethyl-diisopropyl-amin in 700 ml Dimethylformamid 14 h bei 50°C. Nach Einengen des Ansatzes unter vermindertem Druck nahm man den Rückstand mit 400 ml Wasser und 400 ml Dichlormethan auf, stellte mit Natriumbicarbonat unter Rühren den pH = 8 ein und arbeitete mit Dichlormethan und Wasser extraktiv auf. Aus den vereinigten Dichlormethanphasen erhielt man nach Trocknen, Einengen und Umkristallisation des Rückstandes aus Isopropanol 23 g 3-Methyl-6-(2-methylsulfanyl-ethyl)-5,6,7,8-tetrahydro-1H-pyrido[4,3-d]pyrimidin-2,4-dion.

### b) 3-Methyl-6-(2-methylsulfinyl-ethyl)-5,6,7,8-tetrahydro-1H-pyrido[4,3-d]pyrimidin-2,4-dion

Die Lösung von 19,5 g 3-Methyl-6-(2-methylsulfanyl-ethyl)-5,6,7,8-tetrahydro-1H-pyrido[4,3-d]pyrimidin-2,4-dion in 200 ml Ethanol wurde bei 55°C innerhalb 15 min mit 21,8 g Magnesium-monoperoxy-phthalat (85%ig) versetzt und 2 h bei 55°C gerührt. Nach Einstellen von pH = 7,5 wurde unter vermindertem Druck eingeengt. Den Rückstand kochte man mit Methanol mehrfach auf, engte die vereinigten Methanolphasen ein und reinigte den Rückstand durch Chromatographie auf einer Mitteldrucksäule mit Kieselgel und Dichlormethan/Methanol (Volumenverhältnis 95:5) als Laufmittel sowie durch Umkristallisation aus Isopropanol.
Ausbeute: 9,8 g
Zersetzung 228°C

| C₁₁H₁₇N₃O₃S (MG = 271,34) | | | | |
|---|---|---|---|---|
| Ber. | C 48,69 % | H 6,31 % | N 15,49 % | S 11,82 % |
| Gef. | C 48,6 % | H 6,3 % | N 15,4 % | S 11,8 % |

### Beispiel 11

### 6-Benzyl-1-methyl-5,6,7,8-tetrahydro-3H-pyrido[4,3-d]pyrimidin-2,4-dion-hydrochlorid

15,7 g 6-Benzyl-5,6,7,8-tetrahydro-1,3-H-pyrido[4,3-d]pyrimidin-2,4-dion wurden mit 8,7 g Kaliumcarbonat in 700 ml Dimethylformamid 1,5 h bei 50°C gerührt. Man tropfte während 10 min 8,9 g Methyljodid hinzu und rührte weitere 5 h bei 50°C. Nach Einengen der Suspension unter vermindertem Druck arbeitete man den Rückstand mit Wasser und Dichlormethan extraktiv auf. Die getrockneten Dichlormethanphasen wurden eingeengt und der Rückstand zur Abtrennung der weiteren Methylierungsprodukte 6-Benzyl-1,3-dimethyl-5,6,7,8-tetrahydro-pyrido[4,3-d]pyrimidin-2,4-dion und 6-Benzyl-3-methyl-5,6,7,8-tetrahydro-1H-pyrido[4,3-d]pyrimidin-2,4-dion vom angestrebten 6-Benzyl-1-methyl-5,6,7,8-tetrahydro-3H-pyrido[4,3-d]pyrimidin-2,4-dion einer Chromatographie an Kieselgel über eine Mitteldrucksäule mit dem Laufmittelgemisch Dichlormethan/Ethanol (Volumenverhältnis 98:2) unterworfen. Die Fraktionen mit sauberem 6-Benzyl-1-methyl-5,6,7,8-tetrahydro-3H-pyrido[4,3-d]pyrimidin-2,4-dion wurden in Dichlormethan mit etherischer HCl als 6-Benzyl-1-methyl-5,6,7,8-tetrahydro-3H-pyrido[4,3-d]pyrimidin-2,4-dion-hydrochlorid ausgefällt. Nach Rekristallisation aus Wasser erhielt man:
Ausbeute: 2,6 g
Zersetzung 293°C

| C₁₅H₁₈ClN₃O₂ (MG = 307,78) | | | | |
|---|---|---|---|---|
| Ber. | C 58,54 % | H 5,90 % | Cl 11,52 % | N 13,65 % |
| Gef. | C 58,34 % | H 5,92 % | Cl 11,44 % | N 13,58 % |

### Beispiel 12

### 6-(4-Hydroxybutyl)-3-methyl-5,6,7,8-tetrahydro-1H-pyrido[4,3-d]pyrimidin-2,4-dion

### a) 3-Methyl-6-[(4-tetrahydro-pyran-2-yloxy)-butyl]-5,6,7,8-tetrahydro-1H-pyrido[4,3-d]pyrimidin-2,4-dion

Man versetzte 36,3 g 3-Methyl-5,6,7,8-tetrahydro-1H-pyrido[4,3-d]pyrimidin-2,4-dion in 1 l Dimethylformamid unter Rühren mit 27 g Ethyl-diisopropyl-amin, 18 g Lithiumbromid sowie 46 g 2-(4-Chlorbutoxy)-tetrahydro-pyran (86,3%ig gemäß GC) und rührte 5 h bei 70°C. Man engte unter vermindertem Druck ein und erhielt nach extraktiver Aufarbeitung des Rückstandes mit Dichlormethan und Wasser sowie Trocknen und Einengen der gesammelten Dichlormethanphasen 34,5 g rohes 3-Methyl-6-[4-(tetrahydro-pyran-2-yloxy)-butyl]-5,6,7,8-tetrahydro-1H-pyrido[4,3-d]pyrimidin-2,4-dion, das durch Säulenchromatographie an Aluminiumoxid (Aktivitätsstufe III) und Elution mit Dichlormethan/Ethanol (Volumenverhältnis 90:10) gereinigt wurde.

### b) 6-(4-Hydroxybutyl)-3-methyl-5,6,7,8-tetrahydro-1H-pyrido[4,3-d]pyrimidin-2,4-dion

Man löste 12 g 3-Methyl-6-[4-(tetrahydropyran-2-yloxy)-butyl)]-5,6,7,8-tetrahydro-1H-pyrido[4,3-d]pyrimidin-2,4-dion in 150 ml Methanol und 150 ml Wasser und stellte unter Rühren bei 20-25°C mit konz. Salzsäure auf pH = 1. Nach 7 h bei Raumtemperatur wurde unter vermindertem Druck zur Trockne eingeengt, der Rückstand mit Wasser aufgenommen und die Lösung auf pH = 7,8 gestellt. Man engte im Vakuum ein und extrahierte den Rückstand erschöpfend mit heißem Ethanol. Man dampfte den ethanolischen Extrakt unter vermindertem Druck ein und reinigte das rohe 6-(4-Hydroxybutyl)-3-methyl-5,6,7,8-tetrahydro-1H-pyrido-[4,3-d]pyrimidin-2,4-dion durch Säulenchromatographie an Kieselgel mit Dichlormethan/Methanol (Volumenverhältnis 90:10) sowie durch Umkristallisation aus Isopropanol.
Ausbeute: 3,2 g
Schmelzpunkt 121°C

| C₁₂H₁₉N₃O₃ (MG = 253,3) | | | |
|---|---|---|---|
| Ber. | C 56,90 % | H 7,57 % | N 16,59 % |
| Gef. | C 56,65 % | H 7,67 % | N 16,35 % |

### Beispiel 13

### 6-(3-Methyl-2,4-dioxo-2,3,4,5,7,8-hexahydro-1H-pyrido[4,3-d]pyrimidin-6-yl)-hexansäure-hydrochlorid

### a) 6-(3-Methyl-2,4-dioxo-2,3,4,5,7,8-hexahydro-1H-pyrido[4,3-d]pyrimidin-6-yl)-hexansäure-tert.-butylester

Man versetzte 27,2 g 3-Methyl-5,6,7,8-tetrahydro-1H-pyrido[4,3-d]pyrimidin-2,4-dion in 0,5 l Dimethylformamid unter Rühren mit 22 g Ethyl-diisopropyl-amin und 38 g 6-Brom-hexan-säure-tert.-butylester (aus 6-Brom-hexansäure und Isobutylen in Dichlormethan in Gegenwart von konz. Schwefelsäure als Katalysator) und rührte 72 h bei Raumtemperatur. Man engte unter vermindertem Druck ein und erhielt nach extraktiver Aufarbeitung des Rückstandes mit Dichlormethan und Wasser (auf pH = 8 gestellt) sowie Einengen der gesammelten und getrockneten Dichlormethanphasen 55 g öliges Rohprodukt, das durch Umkristallisation aus Diethylether/Petrolether gereinigt wurde. Man erhielt 31 g 6-(3-Methyl-2,4-dioxo-2,3,4,5,7,8-hexahydro-1H-pyrido[4,3-d]pyrimidin-6-yl)-hexansäure-tert.-butylester.

### b) 6-(3-Methyl-2,4-dioxo-2,3,4,5,7,8-hexahydro-1H-pyrido[4,3-d]pyrimidin-6-yl)-hexansäure-hydrochlorid

Die Lösung von 8,3 g 6-(3-Methyl-2,4-dioxo-2,3,4,5,7,8-hexahydro-1H-pyrido[4,3-d]pyrimidin-6-yl)-hexansäure-tert.-butylester in 300 ml Dichlormethan wurde bei 0°C mit HCl-Gas gesättigt und 1 h bei 0°C nachgerührt. Nach Einengen des Ansatzes unter vermindertem Druck nahm man den Rückstand mit Wasser auf und stellte mit Natronlauge auf pH = 8,5. Nach Extraktion mit Dichlormethan wurde die wäßrige Phase durch Chromatographie an einem Ionenaustauscher Amberlyst A 26 (Elution mit 0,25 n HCl) gereinigt. Man erhielt nach Einengen des wäßrigen Eluats 5,9 g 6-(3-Methyl-2,4-dioxo-2,3,4,5,7,8-hexahydro-1H-pyrido[4,3-d]pyrimidin-6-yl)-hexansäure-hydrochlorid, das durch Umkristallisation aus Ethanol/Wasser gereinigt wurde.
Ausbeute: 5 g
Zersetzung 110°C

| C₁₄H₂₂ClN₃O₄ mit 1 H₂O (MG = 349,82) | | | | |
|---|---|---|---|---|
| Ber. | C 48,07 % | H 6,92 % | Cl 10,13 % | N 12,01 % |
| Gef. | C 48,17 % | H 6,73 % | Cl 10,15 % | N 11,96 % |

### Beispiel 14

### 1-(2-Hydroxyethyl)-3,6-dimethyl-5,6,7,8-tetrahydro-1H-pyrido[4,3-d]pyrimidin-2,4-dion

### a) 3,6-Dimethyl-5,6,7,8-tetrahydro-1H-pyrido[4,3-d]pyrimidin-2,4-dion-hydrochlorid

Zu 181 g 3-Methyl-5,6,7,8-tetrahydro-1H-pyrido[4,3-d]pyrimidin-2,4-dion und 178 ml Ethyl-diisopropyl-amin in 3 l DMF tropfte man bei 21°C Anfangstemperatur unter Rühren innerhalb 15 min 63,4 ml Methyljodid (Temperaturerhöhung auf 35°C). Nach weiteren 5 h Rühren bei Raumtemperatur engte man den Ansatz unter vermindertem Druck ein und arbeitete den Rückstand mit Dichlormethan und Wasser extraktiv auf. Die vereinigten Wasserphasen wurden bei 70°C und vermindertem Druck bis zur Niederschlagsbildung eingeengt und der Niederschlag (39,5 g) aus überwiegend 3,6,6-Trimethyl-5,6,7,8-tetrahydro-1H-pyrido[4,3-d]pyrimidin-2,4-dion-hydrojodid abfiltriert. Die Mutterlauge engte man ein und kochte den Rückstand mit 1 l Ethanol auf. Man erhielt weitere 15,7 g unlöslichen Rückstand aus überwiegend 3,6,6-Trimethyl-5,6,7,8-tetrahydro-1H-pyrido[4,3-d]pyrimidin-2,4-dion-hydrojodid. Die vereinigten Niederschläge ergaben nach Reinigung durch Auskochen mit Methanol 45 g reines 3,6,6-Trimethyl-5,6,7,8-tetrahydro-1H-pyrido[4,3-d]pyrimidin-2,4-dion-hydrojodid.
Zersetzung 288°C

| C₁₀H₁₆IN₃O₂ (MG = 337,16) | | | | |
|---|---|---|---|---|
| Ber. | C 35,62 % | H 4,78 % | I 37,64 % | N 12,46 % |
| Gef. | C 35,56 % | H 4,74 % | I 37,86 % | N 12,38 % |

Die methanolischen und ethanolischen Mutterlaugen wurden vereinigt und unter vermindertem Druck eingeengt. Der Rückstand aus überwiegend 3,6-Dimethyl-5,6,7,8-tetrahydro-1H-pyrido[4,3-d]pyrimidin-2,4-dion-hydrojodid wurde durch Chromatographie an einem stark sauren Ionenaustauscher Amberlyst A 15 unter Elution mit 1 n HCL gereinigt. Nach Einengen der entsprechenden Fraktionen und Trocknen des Rückstands erhielt man 55 g 3,6-Dimethyl-5,6,7,8-tetrahydro-1H-pyrido[4,3-d]pyrimidin-2,4-dion-hydrochlorid als weißes Pulver.

### b) 3,6-Dimethyl-1-[2-(tetrahydro-pyran-2-yloxy)-ethyl]-5,6,7,8-tetrahydro-1H-pyrido[4,3-d]pyrimidin-2,4-dion

Man versetzte die Suspension von 3 g Natriumhydrid in 75 ml DMF innerhalb von 5 min mit 11,6 g 3,6-Dimethyl-5,6,7,8-tetrahydro-1H-pyrido[4,3-d]pyrimidin-2,4-dion-hydrochlorid. Dabei stieg die Temperatur auf 60°C an. Nach 1,5 h Rühren bei 60°C tropfte man innerhalb 10 min 11,6 g 2-(2-Bromethoxy)-tetrahydro-pyran zu. Nach weiteren 8 h Rühren bei 60°C wurde der Ansatz unter vermindertem Druck eingeengt und der Rückstand mit Dichlormethan und Wasser extraktiv aufgearbeitet. Die Dichlormethanphasen wurden vereinigt, getrocknet und eingeengt. Aus dem Rückstand erhielt man nach Kugelrohrdestillation bei 50°C und 0,05 mbar sowie Säulenchromatographie an Aluminiumoxid (Aktivitätsstufe III) mit Dichlormethan/Ethanol (Volumenverhältnis 98:2) 3 g 3,6-Dimethyl-1-[2-(tetrahydro-pyran-2-yloxy)-ethyl]-5,6,7,8-tetrahydro-1H-pyrido[4,3-d]pyrimidin-2,4-dion.

### c) 1-(2-Hydroxyethyl)-3,6-dimethyl-5,6,7,8-tetrahydro-1H-pyrido[4,3-d]pyrimidin-2,4-dion

Man löste 3 g 3,6-Dimethyl-1-[2-(tetrahydro-pyran-2-yloxy)-ethyl]-5,6,7,8-tetrahydro-1H-pyrido[4,3-d]pyrimidin-2,4-dion in 150 ml Wasser und stellte bei 20-25°C mit konz. Salzsäure unter Rühren pH = 1 ein. Nach 5 h Rühren bei Raumtemperatur wurde bis pH = 8 2n Natronlauge zugetropft und der Ansatz unter vermindertem Druck eingeengt. Der Rückstand wurde nach azeotroper Trocknung mit Ethanol durch Chromatographie an Kieselgel auf einer Mitteldrucksäule mit Dichlormethan/Methanol (Volumenverhältnis 85:15) und durch Umkristallisation aus Dichlormethan/Diethylether gereinigt.
Ausbeute: 0,7 g
Schmelzpunkt 120 - 122°C

| C₁₁H₁₇N₃O₃ (MG = 239,28) | | | |
|---|---|---|---|
| Ber. | C 55,22 % | H 7,16 % | N 17,56 % |
| Gef. | C 55,02 % | H 7,32 % | N 17,64 % |

### Beispiel 15

### 3-Benzyl-6-methyl-5,6,7,8-tetrahydro-1H-pyrido[4,3-d]pyrimidin-2,4-dion-hydrochlorid

Man versetzte die Mischung von 5,5 ml 90%iger Ameisensäure und 4 ml 37 %igem Formaldehyd bei 0°C unter Rühren mit 6,5 g 3-Benzyl-5,6,7,8-tetrahydro-1H-pyrido[4,3-d]pyrimidin-2,4-dion-hydrochlorid. Nach 8 h Erhitzen bei Rückflußtemperatur fügte man bei Raumtemperatur 26,5 ml 1n Salzsäure hinzu und engte anschließend unter vermindertem Druck ein. Aus dem Rückstand erhielt man nach Umkristallisation aus Isopropanol/Wasser 3-Benzyl-6-methyl-5,6,7,8-tetrahydro-1H-pyrido[4,3-d]pyrimidin-2,4-dion-hydrochlorid.
Ausbeute 5,7 g
Zersetzungspunkt 278°C

| C₁₅H₁₈ClN₃O₂ mit 0,2 H₂O (MG = 311,39) | | | | |
|---|---|---|---|---|
| Ber. | C 57,86 % | H 5,96 % | Cl 11,39 % | N 13,49 % |
| Gef. | C 57,81 % | H 5,72 % | Cl 11,71 % | N 13,45 % |

### Pharmakologische Prüfungen

### A) Antithrombotische Aktivität

Ein wichtiger Faktor in der Genese und dem Verlauf von cerebralen oder peripheren arteriellen Verschlußkrankheiten und anderen für diese Substanzgruppe in Anspruch genommenen Indikationen sind thrombotische Ereignisse. So wurden die erfindungsgemäßen Verbindungen auf Hemmung von Laser - induzierter Thrombose getestet. Diese Untersuchungen erfolgten an weiblichen Sprague - Dawley - Ratten mit einem Körpergewicht von ca. 200 g. Die Tiere wurden mit 0,1 mg Atropinsulfat sc prämediziert und mit 100 mg Ketaminhydrochlorid und 4mg Xylazin pro kg Körpergewicht i.p. anästhetisiert. Zur Untersuchung dienten Arteriolen und Venolen des mit entgastem Paraffinöl überschichteten Mesenteriums mit einem Durchmesser von ca. 13 µm. Der Strahl des 3W Argon-Lasers (Firma Spectra-Physics, Darmstadt) wurde mittels einer Strahladaptions- und Justieranlage koaxial in den invertierten Strahlengang eines Mikroskops (ICM 405, LD® Epiplan 40/o.60, Zeiss, Oberkochen) eingebracht. Die benutzte Wellenlänge betrug 514.5 nm mit einer Leistung oberhalb des Objectivs von 30 mW. Die Expositionszeit pro Einzelschuß dauerte 1/15 sec. Alle Meßvorgänge wurden per Videokamera (®Trinicon-Röhre, Sony Köln) aufgenommen auf auf einem Recorder (®Sony U-matics 3/4") gespeichert. Die Testsubstanzen wurden den Versuchstieren in verschiedenen Dosierungen oral 1 Stunde, bei iv Gabe 10 min vor Versuchsbeginn verabreicht; Kontrolltiere erhielten die gleiche Menge des Placebos. Die Applikation der Substanzen erfolgte als Einzelgabe einmal am Tag bzw. einmal am Tag über mehrere Tage. Zur Auswertung wurde die Zahl der Laserschüsse gezählt, die benötigt wurde, um eine wandständige Thrombose von einer Mindestgröße des halben Gefäßdurchmessers zu erzeugen. Dies bedeutet, je größer die Anzahl der Laserschüsse desto wirksamer sind die Präparate in diesem Test. In Tabelle 1 ist die prozentuale Hemmung der Thrombose angegeben.

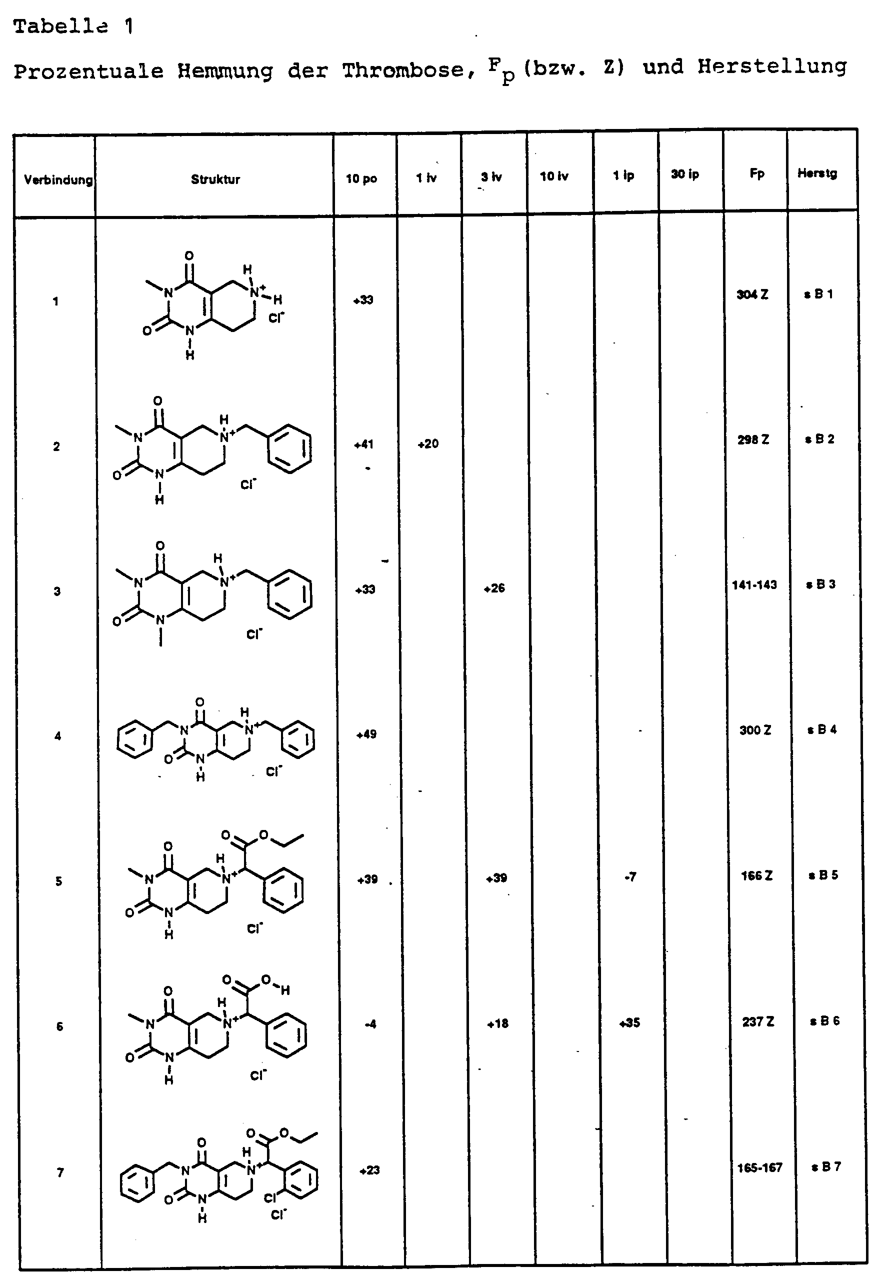

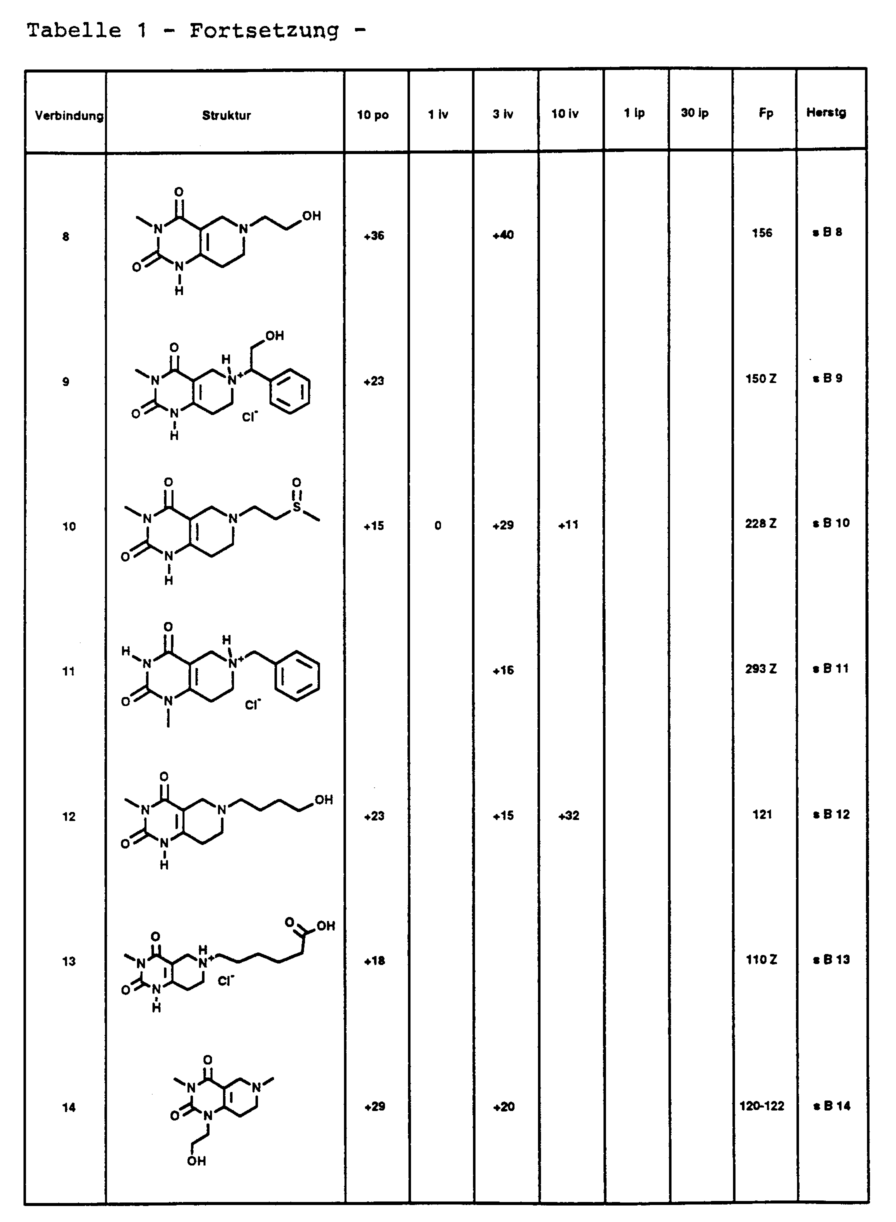

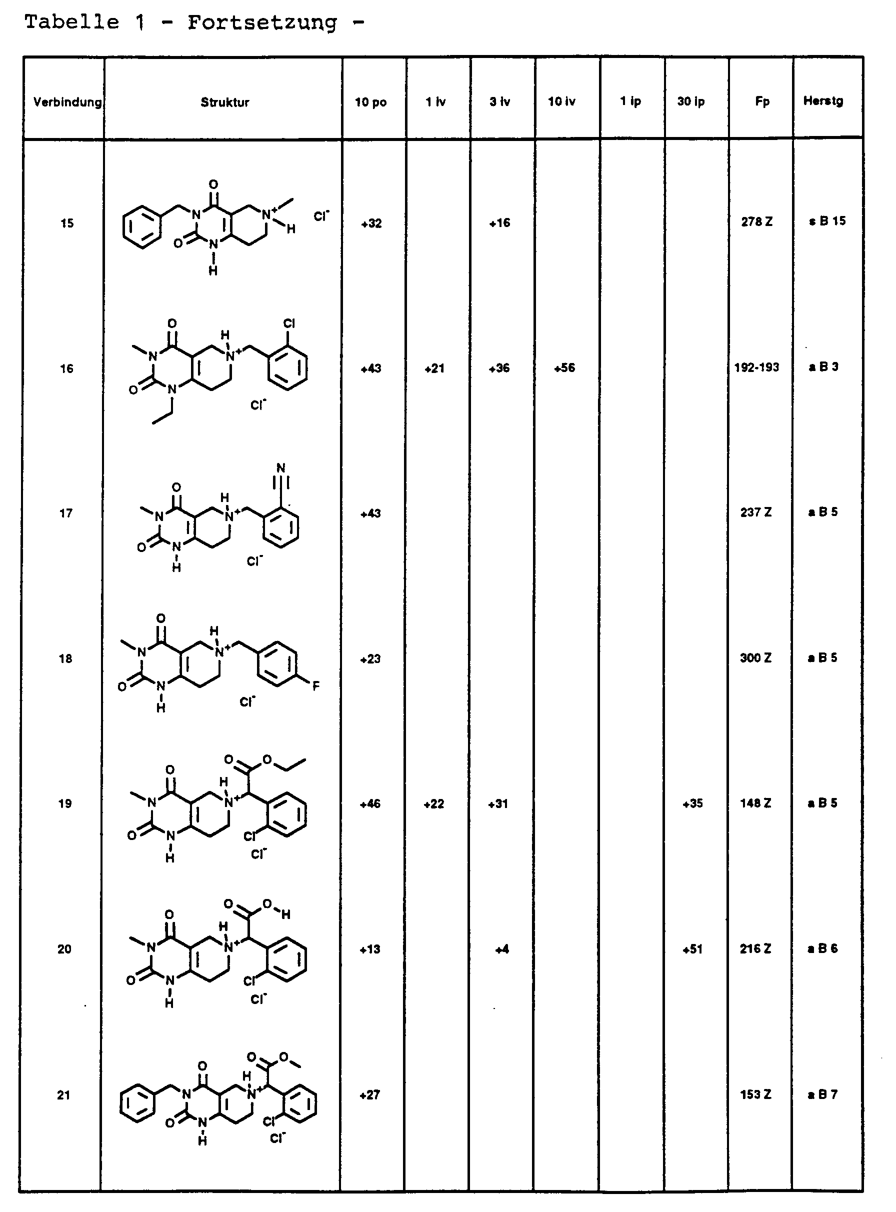

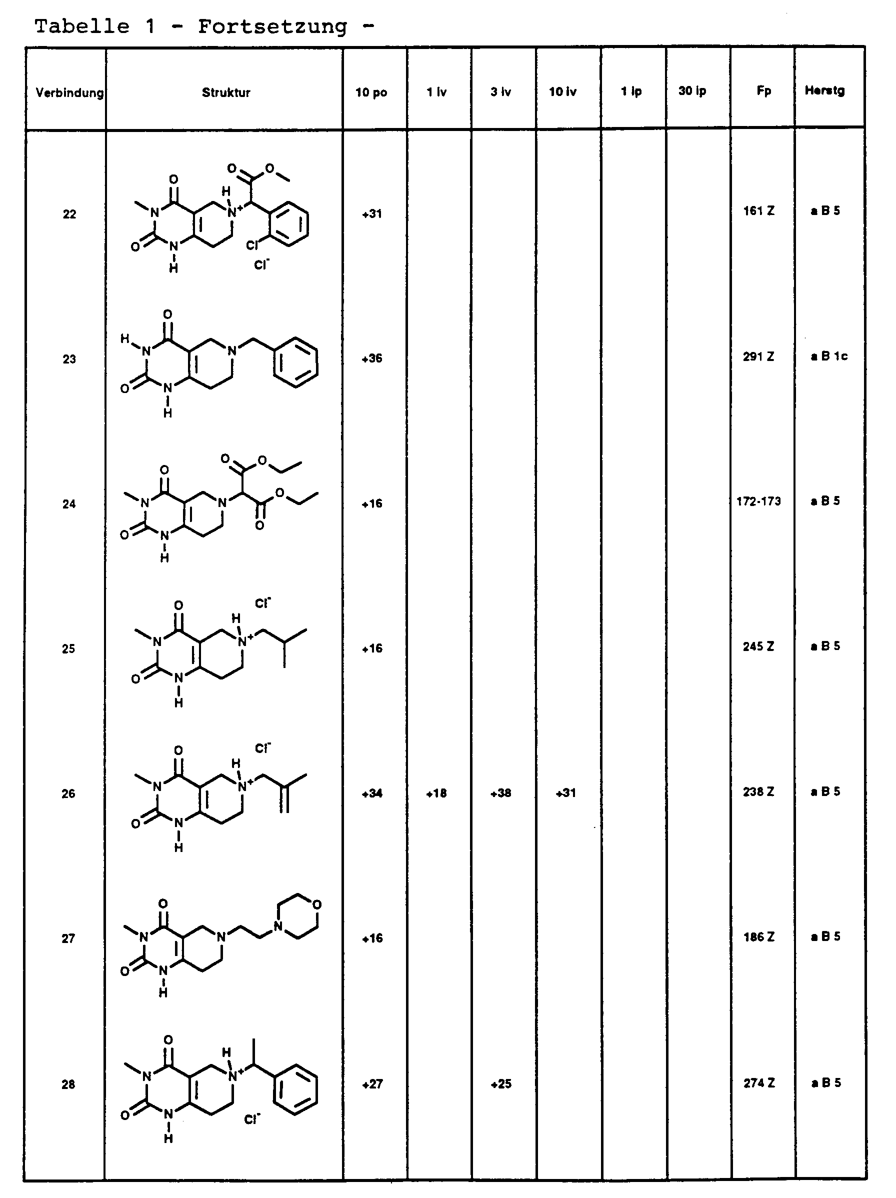

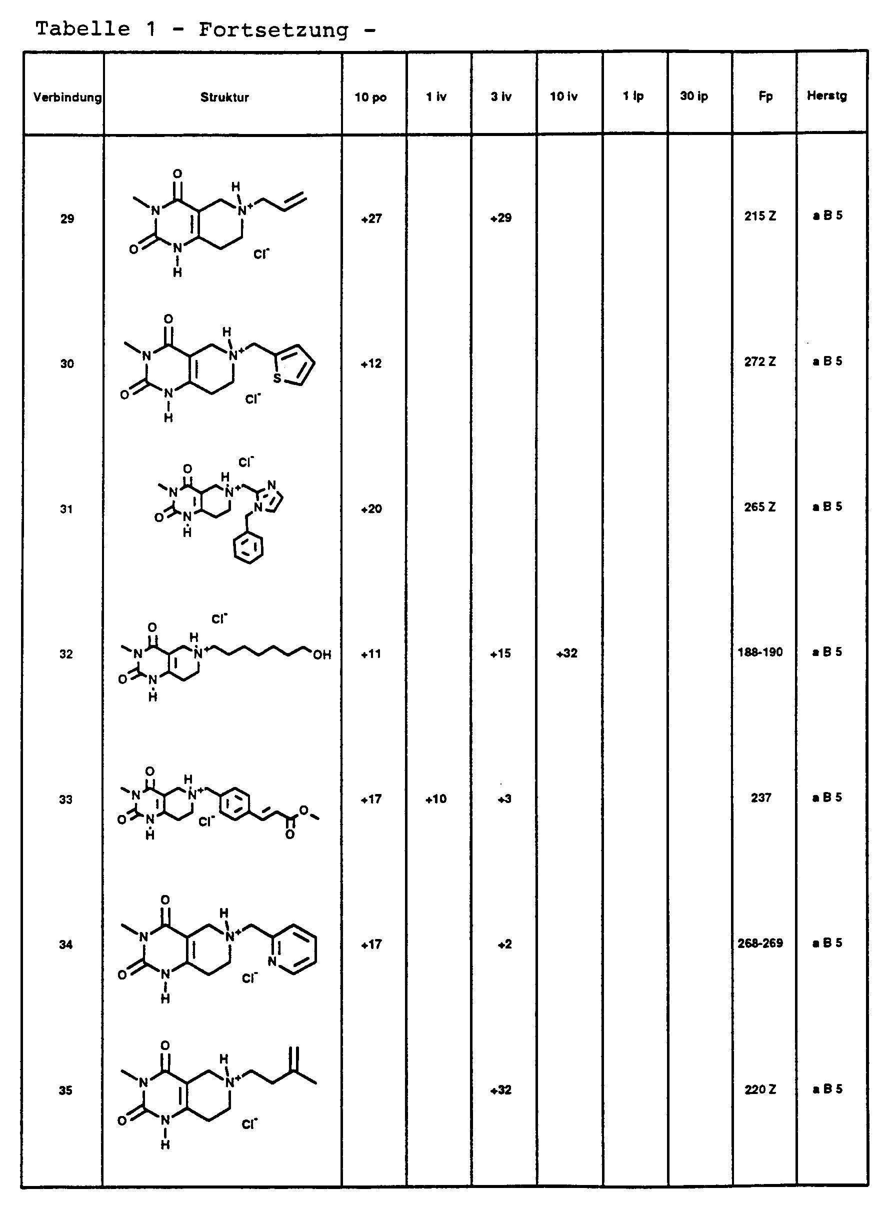

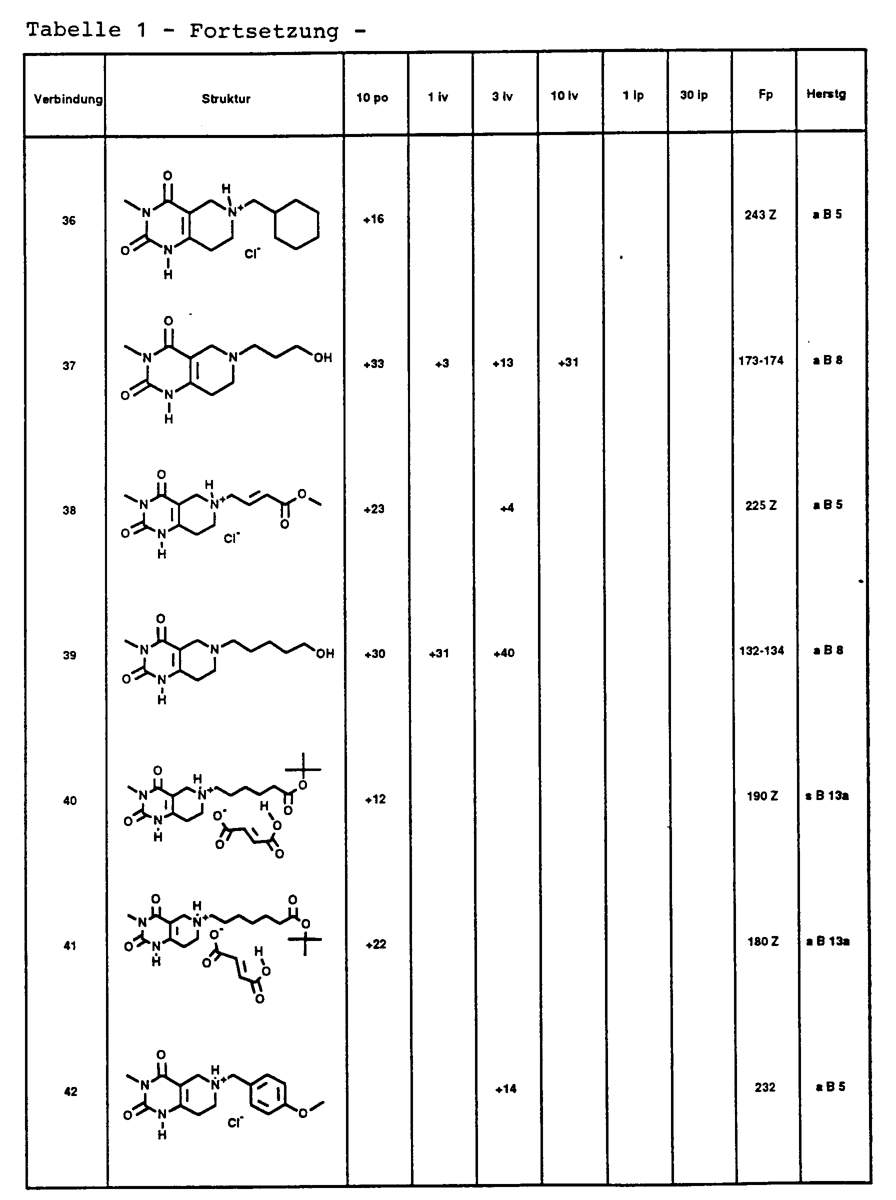

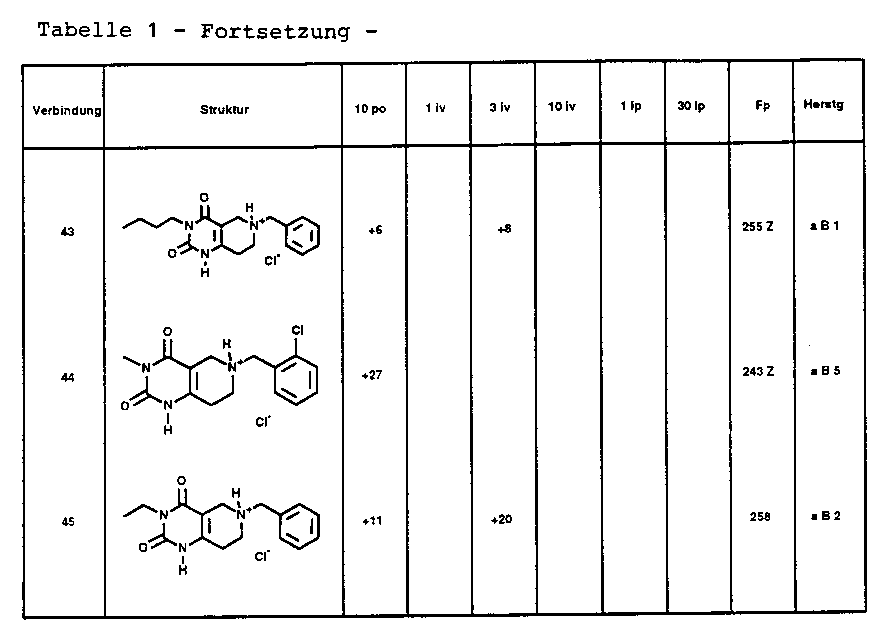

### B) Wirkung auf die Kontraktilität des Skelettmuskels nach chronischer Ischämie

In den letzten Jahren hat sich in den Vorstellungen über die Pathophysiologie der chronischen peripheren arteriellen Verschlußkrankheit ein Wandel vollzogen, als sich das Interesse in zunehmendem Maße von der Makrozirkulation hin zu Mikrozirkulation verlagerte. Störungen in der Mikrozirkulation manifestieren sich demzufolge in einer Unterversorgung mit Substraten mit daraus resultierender Gewebsischämie, die wiederum zu einer Beeinträchtigung der Funktion der betroffenen Extremität führt. Die Prüfung der erfindungsgemäßen Verbindungen auf ihre die Funktion verbessernde Wirkung erfolgte anhand von Messungen der Kontraktionskraft im ischämischen Skelettmuskel mit der unten beschriebenen Versuchsanordnung.

Als Versuchstiere dienten männliche Wistar-Ratten mit einem Körpergewicht von 380 bis 410 g. In Hexobarbital Narkose (®Evipan-Natrium, 200 mg/kg Körpergewicht i.p.) wurde den Tieren eine einseitige Ligatur der rechten Femoralarterie in der Leiste gesetzt. Nach Einstreuen von Penizillin-Sulfonamid-Puder zur antibiotischen Wundversorgung wurde die kleine Operationswunde verschlossen und die Tiere wurden bis zum völligen Erwachen ständig observiert. Eine Woche später begann die Substanzapplikation durch orale Gabe mit einer Schlundsonde (6 mg/kg Körpergewicht, CarboxymethylcelluloseNatrium-Suspension) und wurde für 7 Tage weitergeführt (Einmalgabe pro Tag, ca. 7h30 bis 8h30). Die Kontraktionskraft wurde 24 h nach der letzten Substanzgabe gemessen, um akute Effekte auszuschließen, und zwar nach folgendem Versuchsprotokoll:
Die Tiere wurden mit ®Nembutal (Pentobarbital-Natrium, 35 mg/kg Körpergewicht i.p.) narkotisiert, die Muskeln der betreffenden Extremität freigelegt (Gastrocnemius-Plantaris-Soleus-Gruppe) und die Sehne wurde an einen Kraftaufnehmer mit einer Vorlast von 50 g angebunden. Eine Superfusion mit physiologischer Kochsalzlösung (37°C) diente zur Vermeidung von Austrocknung und Abkühlung. Der mittlere arterielle Blutdruck wurde mit Hilfe eines Blutdruckmeßgeräts über eine kanülierte Karotisarterie kontinuierlich zur Kontrolle des physiologischen Status der Tiere während des Versuchs aufgezeichnet. Alle Tiere atmeten spontan durch einen eingeführten Trachealtubus.

Nach diesen Vorbereitungen wurde der Muskel durch direkte elektrische Stimulation (2.5 mA, 2 Hz) zur Kontraktion gebracht (Stimulator I, Firma Hugo Sachs, Bundesrepublik Deutschland). Als Meßparameter für die Präparatewirkung diente die absolute Kontraktionskraft in Gramm nach 5 Minuten während der Stimulation. Die Änderung der Kontraktionskraft im Vergleich zum ischämischen Kontrollmuskel ist als Prozentänderung zu diesem Zeitpunkt (s. Tabelle 2) angegeben.

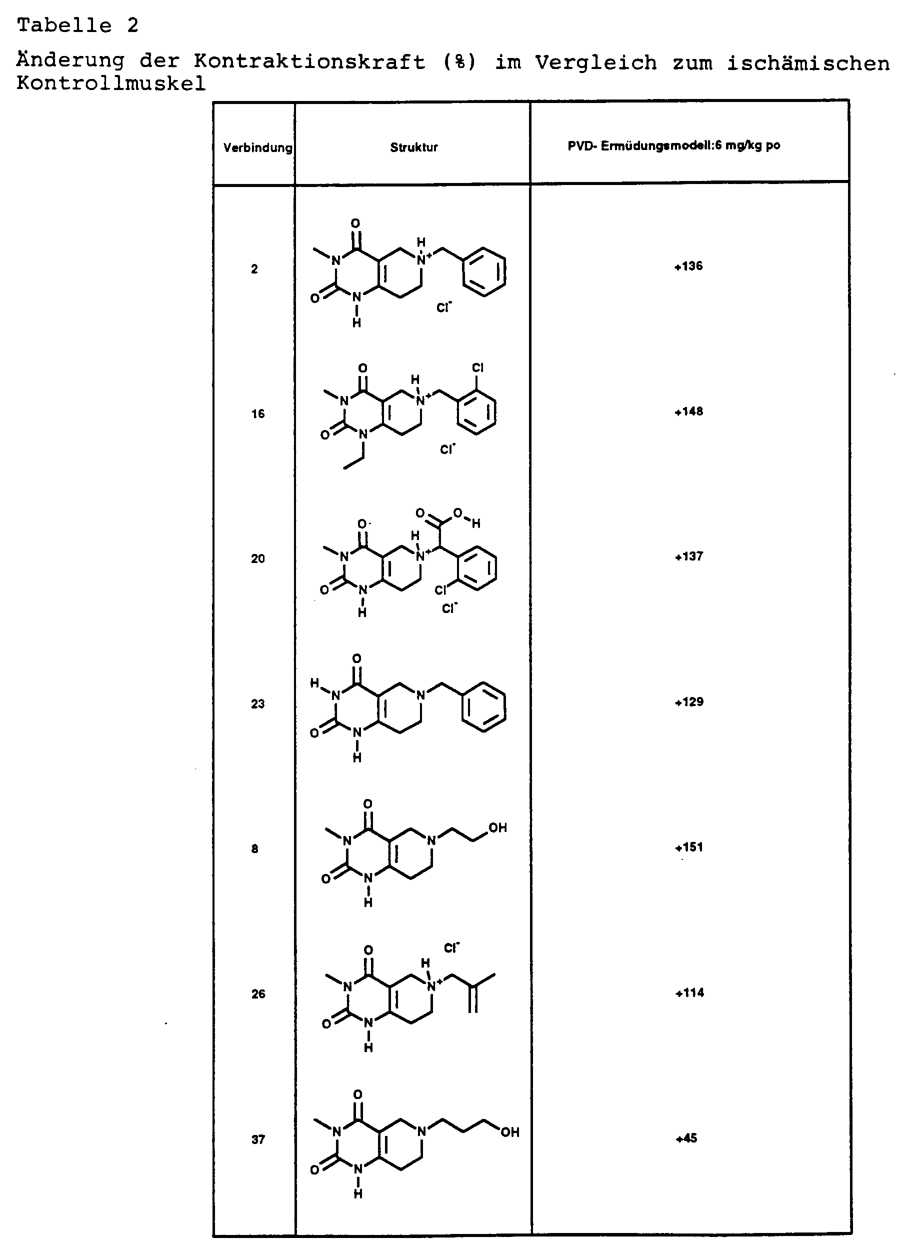

## Patentansprüche

1. Verwendung von mindestens einer Verbindung der Formel I 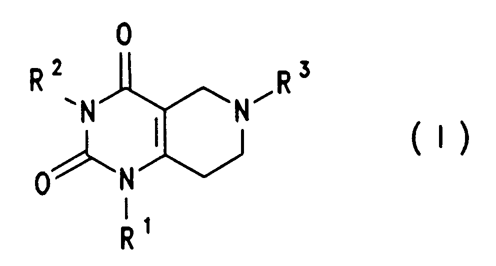 und/oder eines physiologisch verträglichen Salzes der Verbindung der Formel I und/oder einer gegebenenfalls stereoisomeren Form der Verbindung der Formel I, wobei
R¹ für
a) Wasserstoffatom,
b) (C₁-C₄)-Alkyl,
c) ω-Hydroxy-(C₂-C₄)-alkyl,
d) Benzyl,
e) Benzyl, ein- oder mehrfach am Kern substituiert durch
1) Halogenatom wie Fluor-, Chlor-, Brom- oder Jodatom,
2) Nitril oder
3) Methoxy,
f) (ω-1)-(C₃-C₅)-Alkenyl oder
g) (ω-1)-(C₃-C₅)-Alkenyl,substituiert am (ω-1)-Kohlenstoff durch Methyl, steht,
R² für
a) Wasserstoffatom,
b) (C₁-C₆)-Alkyl,
c) Benzyl oder
d) Benzyl, ein- oder mehrfach am Kern substituiert durch
1) Halogenatom wie Fluor-, Chlor-, Brom- oder Jodatom,
2) Nitril oder
3) Methoxy, steht und
R³ für
a) Wasserstoffatom,
b) (C₁-C₈)-Alkyl,
c) Cyclohexyl-methyl,
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
1.1 Halogenatom wie Fluor-, Chlor-, Brom- oder Jodatom,
1.2 Nitril,
1.3 Methoxy oder
1.4 -CH=CH-COOR⁴, worin R⁴ Wasserstoffatom oder (C₁-C₄)-Alkyl bedeutet,
und/oder 2) in α-Position des Benzylrestes einfach substituiert durch
2.1 Methyl,
2.2 Hydroxymethyl,
2.3 Carboxyl oder
2.4 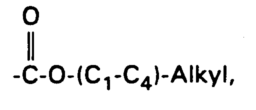
i) -(CH₂)ₙ-COOR⁵, worin
n eine ganze Zahl von 1 bis 8 darstellt und
R⁵
a) Wasserstoffatom oder
b) (C₁-C₄)-Alkyl bedeutet,
k) -CH₂-CH=CH-COOR⁶, worin R⁶ (C₁-C₄)-Alkyl bedeutet,
l) Pyridylmethyl,
m) 1-Benzyl-imidazol-2-yl-methyl,
n) -CH(COOR⁶)₂, worin R⁶ (C₁-C₄)-Alkyl bedeutet,
o) ω-Morpholin-4-yl-(C₂-C₄)-alkyl,
p) 2-Methylsulfinyl-ethyl oder
q) Thienyl-methyl (Thenyl)
steht, zur Herstellung von Arzneimitteln zur Prophylaxe und Therapie von Durchblutungsstörungen und/oder neurodegenerativen Erkrankungen.

2. Verwendung nach Anspruch 1, dadurch gekennzeichnet, daß man mindestens eine Verbindung der Formel I, wobei
R¹ für
a) Wasserstoffatom,
b) (C₁-C₄)-Alkyl,
c) ω-Hydroxy-(C₂-C₄)-alkyl,
d) Benzyl,
e) Benzyl, ein- oder mehrfach am Kern substituiert durch
1) Fluor- oder Chloratom,
2) Nitril oder
3) Methoxy,
f) (ω-1)-(C₃-C₅)-Alkenyl oder
g) (ω-1)-(C₃-C₅)-Alkenyl, substituiert am (ω-1)-Kohlenstoff durch Methyl, steht,
R² für
a) Wasserstoffatom
b) (C₁-C₆)-Alkyl,
c) Benzyl oder
d) Benzyl, ein- oder mehrfach am Kern substituiert durch
1) Fluor- oder Chloratom,
2) Nitril oder
3) Methoxy, steht und
R³ für
a) Wasserstoffatom,
b) (C₁-C₈)-Alkyl,
c) Cyclohexyl-methyl,
d) ω-Hydroxy-(C₂-C₈)-alkyl,
e) (ω-)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
1.1 Fluor- oder Chloratom,
1.2 Nitril,
1.3 Methoxy oder
1.4 -CH=CH-COOR⁴, worin R⁴ Wasserstoffatom oder (C₁-C₄)-Alkyl bedeutet,
und/oder 2) in α-Position des Benzylrestes einfach substituiert durch
2.1 Methyl,
2.2 Hydroxymethyl,
2.3 Carboxyl,
2.4 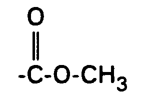 oder
2.5 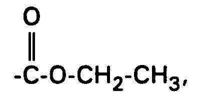
i) -(CH₂)ₙ-COOR⁵, worin n eine ganze Zahl von 1 bis 8 darstellt und
R⁵
a) Wasserstoffatom oder
b) (C₁-C₄)-Alkyl bedeutet,
k) -CH₂-CH=CH-COOR⁶, worin R⁶ (C₁-C₄)-Alkyl bedeutet,
l) 2-Pyridylmethyl,
m) 1-Benzyl-imidazol-2-yl-methyl,
n) -CH(COOR⁶)₂, worin R⁶ (C₁-C₄)-Alkyl bedeutet,
o) ω-Morpholin-4-yl-(C₂-C₄)-alkyl,
p) 2-Methylsulfinyl-ethyl oder
q) 2-Thienyl-methyl
steht, einsetzt.

3. Verbindung der Formel I 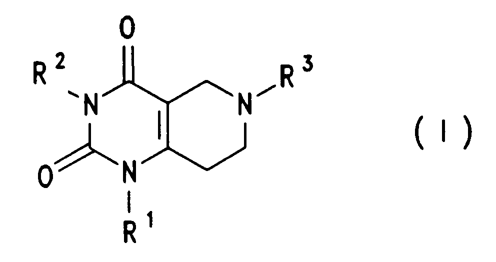 und physiologisch verträgliche Salze der Verbindung der Formel I und gegebenenfalls stereoisomere Formen der Verbindung der Formel I, wobei
R¹ für
a) Wasserstoffatom,
b) (C₁-C₄)-Alkyl,
c) ω-Hydroxy-(C₂-C₄)-alkyl,
d) Benzyl,
e) Benzyl, ein- oder mehrfach am Kern substituiert durch
1) Halogenatom wie Fluor-, Chlor-, Brom- oder Jodatom
2) Nitril oder
3) Methoxy,
f) (ω-1)-(C₃-C₅)-Alkenyl oder
g) (ω-1)-(C₃-C₅)-Alkenyl, substituiert am (ω-1)-Kohlenstoff durch Methyl, steht,
R² für
a) Wasserstoffatom,
b) (C₁-C₆)-Alkyl,
c) Benzyl oder
d) Benzyl, ein- oder mehrfach am Kern substituiert durch
1) Fluor-, Chlor-, Brom- oder Jodatom,
2) Nitril oder
3) Methoxy steht und
R³ für
a) Wasserstoffatom,
b) (C₁-C₈)-Alkyl,
c) Cyclohexyl-methyl,
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
1.1 Fluor- oder Chloratom,
1.2 Nitril,
1.3 Methoxy,
1.4 -CH=CH-COOR⁴, worin R⁴ Wasserstoffatom oder (C₁-C₄)-Alkyl bedeutet,
und/oder 2) in α-Position des Benzylrestes einfach substituiert durch
2.1 Methyl,
2.2 Hydroxymethyl,
2.3 Carboxyl,
2.4 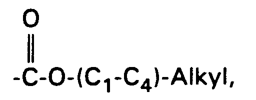
i) -(CH₂)ₙ-COOR⁵, worin
n eine ganze Zahl von 1 bis 8 darstellt und
R⁵
a) Wasserstoffatom oder
b) (C₁-C₄)-Alkyl bedeutet,
k) -CH₂-CH=CH-COOR⁶, worin R⁶ (C₁-C₄)-Alkyl bedeutet,
l) Pyridylmethyl,
m) 1-Benzyl-imidazol-2-yl-methyl,
n) -CH(COOR⁶)₂, worin R⁶ (C₁-C₄)-Alkyl bedeutet,
o) ω-Morpholin-4-yl-(C₂-C₄)-alkyl,
p) 2-Methylsulfinyl-ethyl oder
q) Thienyl-methyl steht,
wobei die Verbindung der Formel I, worin R¹ und R² Wasserstoffatom sind, ausgenommen ist.

4. Verbindung der Formel I nach Anspruch 3, wobei
R¹ für
a) Wasserstoffatom,
b) (C₁-C₄)-Alkyl,
c) 2-Hydroxyethyl,
d) Benzyl oder
e) 2-Methylallyl, steht,
R² für
a) Wasserstoffatom,
b) (C₁-C₆)-Alkyl oder
c) Benzyl steht und
R³ für
a) Wasserstoffatom,
b) (C₁-C₈)-Alkyl,
c) Cyclohexyl-methyl
d) ω-Hydroxy-(C₂-C₈)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
1.1 Halogenatom wie Fluor- oder Chloratom,
1.2 Nitril,
1.3 Methoxy,
1.4 -CH=CH-COOR⁴, worin R⁴ Methyl oder Ethyl bedeutet,
und/oder 2) in α-Position des Benzylrestes einfach substituiert durch
2.1 Methyl,
2.2 Hydroxymethyl,
2.3 Carboxyl,
2.4 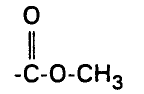 oder
2.5 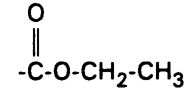 steht,
i) -(CH₂)ₙ-COOR⁵, worin
n eine ganze Zahl von 1 bis 8 darstellt und
R⁵
a) Wasserstoffatom oder
b) (C₁-C₂)-Alkyl bedeutet,
k) -CH₂-CH=CH-COOR⁶, worin R⁶ (C₁-C₂)-Alkyl bedeutet,
l) 2-Pyridylmethyl,
m) 1-Benzyl-imidazol-2-yl-methyl,
n) -CH(COOR⁶)₂, worin R⁶ (C₁-C₂)-Alkyl bedeutet,
o) ω-Morpholin-4-ylethyl,
p) 2-Methylsulfinyl-ethyl oder
q) 2-Thienyl-methyl steht,
wobei die Verbindung der Formel I, worin R¹ und R² Wasserstoffatom sind, ausgenommen ist.

5. Verbindung der Formel I nach Anspruch 3 oder 4, wobei
R¹ für
a) Wasserstoffatom,
b) Methyl oder
c) Ethyl steht,
R² für
a) Wasserstoffatom,
b) (C₁-C₄)-Alkyl oder
c) Benzyl steht,
R³ für
a) Wasserstoffatom,
b) (C₁-C₈)-Alkyl,
c) Cyclohexyl-methyl
d) ω-Hydroxy-(C₂-C₄)-alkyl,
e) (ω-1)-(C₃-C₅)-Alkenyl,
f) (ω-1)-(C₃-C₅)-Alkenyl, substituiert am (ω-1)-Kohlenstoff durch Methyl,
g) Benzyl,
h) Benzyl, 1) ein- oder mehrfach am Kern substituiert durch
1.1 Fluor- oder Chloratom,
1.2 Nitril,
1.3 Methoxy,
1.4 -CH=CH-COOR⁴ am Benzolring, worin R⁴ Methyl oder Ethyl bedeutet,
und/oder 2) in α-Position des Benzylrestes einfach substituiert durch
2.1 Carboxyl,
2.2 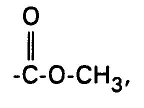
2.3 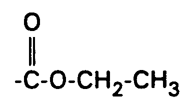
oder
i) 2-Thienyl-methyl steht,
wobei die Verbindung der Formel I, worin R¹ und R² Wasserstoffatom sind, ausgenommen ist.

6. Verfahren zur Herstellung der Verbindung der Formel I nach einem oder mehreren der Ansprüche 3 bis 5, dadurch gekennzeichnet, daß man
A) eine Verbindung der Formel II 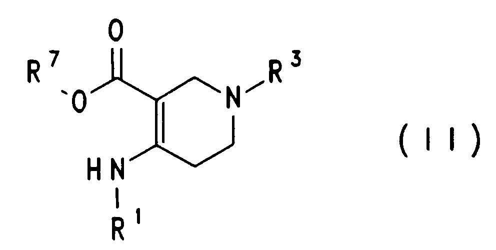 wobei
R¹
a) Wasserstoffatom oder
b) (C₁-C₄)-Alkyl ist,
R³
a) (C₁-C₈)-Alkyl,
b) Cyclohexyl-methyl,
c) Benzyl, ein- oder mehrfach am Kern mit Halogenatom oder Methoxy substituiert wie in Formel I nach Anspruch 1 oder
d) Benzyl ist und
R⁷ einen niederen Alkylrest, vorzugsweise Methyl oder Ethyl, bedeutet, mit einer Verbindung der Formel III
R² - N = C = Y (III)
wobei
R²
a) (C₁-C₆)-Alkyl,
b) Benzyl, ein- oder mehrfach am Kern mit Halogenatom oder Methoxy substituiert wie in Formel I nach Anspruch 1, oder
c) Benzyl ist und
Y Sauerstoffatom bedeutet,
zu einer Verbindung der Formel I umsetzt 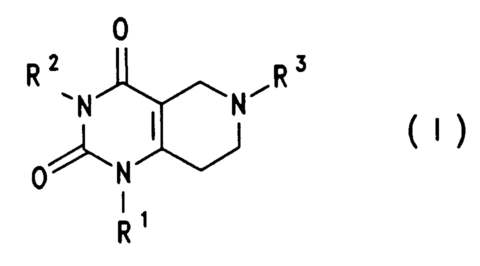 wobei R² die in Formel III sowie R¹ und R³ die in Formel II angegebene Bedeutung haben, oder
B) eine Verbindung der Formel II, wobei R¹, R³ und R⁷ die in Formel II angegebene Bedeutung haben, mit einer Verbindung der Formel III, wobei
R²
a) (C₁-C₆)-Alkyl,
b) Benzyl, ein- oder mehrfach am Kern mit Halogen oder Methoxy substituiert wie in Formel I nach Anspruch 1, oder
c) Benzyl ist und
Y Schwefelatom bedeutet,
zu einer Verbindung der Formel IV umsetzt 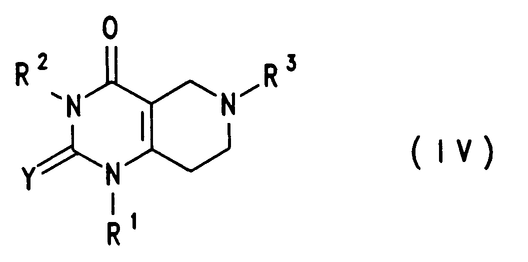 wobei Y Schwefelatom bedeutet und R² die in Formel III sowie R¹ und R³ die in Formel II angegebene Bedeutung haben,
und die Verbindung der Formel IV zu einer Verbindung der Formel I oxidiert, wobei R² die in Formel III sowie R¹ und R³ die in Formel II angegebene Bedeutung haben, oder
C) eine Verbindung der Formel I, wobei R¹ für Wasserstoffatom, (C₁-C₄)-Alkyl, ω-Hydroxy-(C₂-C₄)-Alkyl und R² für Wasserstoffatom, (C₁-C₆)-Alkyl, Benzyl, Benzyl ein- oder mehrfach am Kern substituiert durch Methoxy steht und R³ ein Benzylrest ist zu einer Verbindung der Formel I umsetzt, wobei R³ für Wasserstoffatom steht und R¹ und R² die genannte Bedeutung haben, oder
D) eine Verbindung der Formel I und/oder Salze der Verbindung der Formel I, wobei R¹ und/oder R³ Wasserstoffatom bedeuten und R² die in Formel I angegebene Bedeutung außer Wasserstoffatom hat, mit einer Verbindung der Formel VII
R⁸ - X (VII)
wobei
X für
a) Halogenatom wie Chlor-, Jod- oder Bromatom,
b) Sulfonsäureesterrest oder
c) Phosphorsäureesterrest und
R⁸ die in Formel I für R¹ und R³ angegebene Bedeutung hat, ausgenommen die Bedeutung Wasserstoffatom,
wobei man diejenigen Reste R⁸, die eine alkoholische Hydroxylgruppe oder eine Carboxylgruppe tragen, gegebenenfalls in geschützter Form umsetzt und nach Abspaltung der Schutzgruppe in eine Verbindung der Formel I überführt, oder
E) eine Verbindung der Formel I, wobei R¹ und R² die in Formel I genannte Bedeutung haben mit Ausnahme von (ω-1)-(C₃-C₅)-Alkenyl mit Wasserstoffatom oder Methyl am (ω-1)-Kohlenstoffatom oder Benzyl, ein- oder mehrfach am Kern substituiert durch Nitril, Brom- oder Jodatom und R³ 2-Methylsulfanyl-ethyl ist, mit einem Oxidationsmittel, vorzugsweise einer Persäure, zu einer Verbindung der Formel I umsetzt, wobei R¹ und R² die genannte Bedeutung haben und R³ 2-Methylsulfinylethyl ist, oder
F) eine Verbindung der Formel I, wobei R¹ und R² für Wasserstoffatom, Alkyl oder Benzyl gemäß Formel I stehen und R³ Wasserstoffatom ist, mit einer Verbindung der Formel V 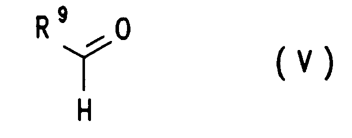 wobei
R⁹
a) Wasserstoffatom,
b) (C₁-C₇)-Alkyl,
c) Cyclohexyl oder
d) Phenyl bedeutet,
unter reduktiven Reaktionsbedingungen zu einer Verbindung der Formel I umsetzt.

7. Arzneimittel, enthaltend eine wirksame Menge von mindestens einer Verbindung der Formel I nach einem oder mehreren der Ansprüche 3 bis 5, oder wie erhalten nach Anspruch 6 und/oder mindestens ein physiologisch verträgliches Salz einer Verbindung der Formel I neben physiologisch annehmbaren Hilfs- und Trägerstoffen, gegebenenfalls weiteren Zusatzstoffen und/oder anderen Wirkstoffen.

8. Arzneimittel nach Anspruch 7 zur Prophylaxe und Therapie von Durchblutungsstörungen und/oder neurodegenerativer Erkrankungen.

9. Verwendung von mindestens einer Verbindung der Formel I nach einem oder mehreren der Ansprüche 3 bis 5 zur Herstellung von Arzneimitteln zur Prophylaxe und Therapie von Durchblutungsstörungen und/oder neurodegenerativer Erkrankungen.

10. Verfahren zur Herstellung eines Arzneimittels nach Anspruch 7 oder 8 dadurch gekennzeichnet, daß man mindestens eine Verbindung der Formel I und/oder mindestens ein physiologisch verträgliches Salz einer solchen Verbindung und/oder eine nach dem Verfahren nach Anspruch 6 erhaltene Verbindung mit physiologisch annehmbaren Hilfs- und Trägerstoffen und gegebenenfalls weiteren Zusatzstoffen und/oder anderen Wirkstoffen in eine geeignete Darreichungsform bringt.

## Claims

1. The use of at least one compound of the formula 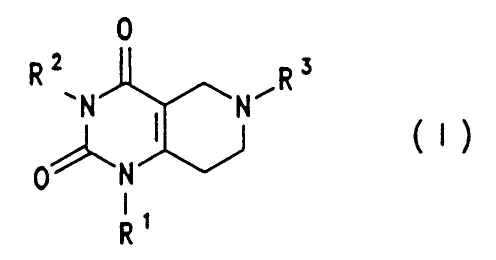 and/or a physiologically acceptable salt of the compound of the formula I and/or an optionally stereoisomeric form of the compound of the formula I in which
R¹ is
a) a hydrogen atom,
b) (C₁-C₄)-alkyl,
c) ω-hydroxy-(C₂-C₄)-alkyl,
d) benzyl,
e) benzyl monosubstituted or polysubstituted on the ring by
1) a halogen atom such as a fluorine, chlorine, bromine or iodine atom,
2) nitrile or
3) methoxy,
f) (ω-1)-(C₃-C₅)-alkenyl or
g) (ω-1)-(C₃-C₅)-alkenyl substituted on the (ω-1) carbon by methyl,
R² is
a) a hydrogen atom,
b) (C₁-C₆)-alkyl,
c) benzyl or
d) benzyl monosubstituted or polysubstituted on the ring by
1) a halogen atom such as a fluorine, chlorine, bromine or iodine atom,
2) nitrile or
3) methoxy and
R³ is
a) a hydrogen atom,
b) (C₁-C₈)-alkyl,
c) cyclohexylmethyl,
d) ω-hydroxy-(C₂-C₈)-alkyl,
e) (ω-1)-(C₃-C₅)-alkenyl,
f) (ω-1)-(C₃-C₅)-alkenyl substituted on the (ω-1) carbon by methyl,
g) benzyl,
h) benzyl 1) monosubstituted or polysubstituted on the ring by
1.1 a halogen atom such as a fluorine, chlorine, bromine or iodine atom,
1.2 nitrile,
1.3 methoxy or
1.4 -CH=CH-COOR⁴ in which R⁴ is a hydrogen atom or (C₁-C₄)-alkyl,
and/or 2) monosubstituted in the a-position of the benzyl radical by
2.1 methyl,
2.2 hydroxymethyl,
2.3 carboxyl or
2.4 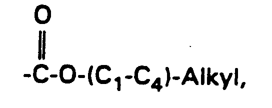
i) -(CH₂)ₙ-COOR⁵ in which
n is an integer from 1 to 8 and
R⁵ is
a) a hydrogen atom or
b) (C₁-C₄)-alkyl,
k) -CH₂-CH=CH-COOR⁶ in which R⁶ is (C₁-C₄)-alkyl,
l) pyridylmethyl,
m) 1-benzylimidazol-2-ylmethyl,
n) -CH(COOR⁶)₂ in which R⁶ is (C₁-C₄)-alkyl,
o) ω-morpholin-4-yl-(C₂-C₄)-alkyl,
p) 2-methylsulfinylethyl or
q) thienylmethyl (thenyl),
for the preparation of pharmaceuticals for the prophylaxis and therapy of circulatory disorders and/or neurodegenerative diseases.

2. The use as claimed in claim 1, which comprises using at least one compound of the formula I in which
R¹ is
a) a hydrogen atom,
b) (C₁-C₄)-alkyl,
c) ω-hydroxy-(C₂-C₄)-alkyl,
d) benzyl,
e) benzyl monosubstituted or polysubstituted on the ring by
1) a fluorine or chlorine atom,
2) nitrile or
3) methoxy,
f) (ω-1)-(C₃-C₅)-alkenyl or
g) (ω-1)-(C₃-C₅)-alkenyl substituted on the (ω-1) carbon by methyl,
R² is
a) a hydrogen atom,
b) (C₁-C₆)-alkyl,
c) benzyl or
d) benzyl monosubstituted or polysubstituted on the ring by
1) a fluorine or chlorine atom,
2) nitrile or
3) methoxy and
R³ is
a) a hydrogen atom,
b) (C₁-C₈)-alkyl,
c) cyclohexylmethyl,
d) ω-hydroxy-(C₂-C₈)-alkyl,
e) (ω-1)-(C₃-C₅)-alkenyl,
f) (ω-1)-(C₃-C₅)-alkenyl substituted on the (ω-1) carbon by methyl,
g) benzyl,
h) benzyl 1) monosubstituted or polysubstituted on the ring by
1.1 a fluorine or chlorine atom,
1.2 nitrile,
1.3 methoxy or
1.4 -CH=CH-COOR⁴ in which R⁴ is a hydrogen atom or (C₁-C₄)-alkyl,
and/or 2) monosubstituted in the a-position of the benzyl radical by
2.1 methyl,
2.2 hydroxymethyl,
2.3 carboxyl,
2.4 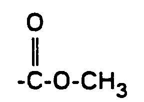 or
2.5 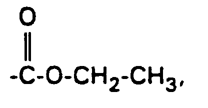
i) -(CH₂)ₙ-COOR⁵ in which
n is an integer from 1 to 8 and
R⁵ is
a) a hydrogen atom or
b) (C₁-C₄)-alkyl,
k) -CH₂-CH=CH-COOR⁶ in which R⁶ is (C₁-C₄)-alkyl,
l) 2-pyridylmethyl,
m) 1-benzylimidazol-2-ylmethyl,
n) -CH(COOR⁶)₂ in which R⁶ is (C₁-C₄)-alkyl,
o) ω-morpholin-4-yl-(C₂-C₄)-alkyl,
p) 2-methylsulfinylethyl or
q) 2-thienylmethyl.

3. A compound of the formula I 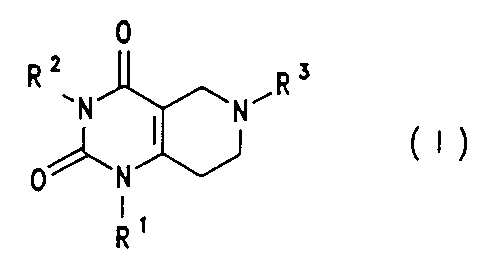 and physiologically acceptable salts of the compound of the formula I and optionally stereoisomeric forms of the compound of the formula I in which
R¹ is
a) a hydrogen atom,
b) (C₁-C₄)-alkyl,
c) ω-hydroxy-(C₂-C₄)-alkyl,
d) benzyl,
e) benzyl monosubstituted or polysubstituted on the ring by
1) a halogen atom such as a fluorine, chlorine, bromine or iodine atom,
2) nitrile or
3) methoxy,
f) (ω-1)-(C₃-C₅)-alkenyl or
g) (ω-1)-(C₃-C₅)-alkenyl substituted on the (ω-1) carbon by methyl,
R² is
a) a hydrogen atom,
b) (C₁-C₆)-alkyl,
c) benzyl or
d) benzyl monosubstituted or polysubstituted on the ring by
1) a fluorine, chlorine, bromine or iodine atom,
2) nitrile or
3) methoxy and
R³ is
a) a hydrogen atom,
b) (C₁-C₈)-alkyl,
c) cyclohexylmethyl,
d) ω-hydroxy-(C₂-C₈)-alkyl,
e) (ω-1)-(C₃-C₅)-alkenyl,
f) (ω-1)-(C₃-C₅)-alkenyl substituted on the (ω-1) carbon by methyl,
g) benzyl,
h) benzyl 1) monosubstituted or polysubstituted on the ring by
1.1 a fluorine or chlorine atom,
1.2 nitrile,
1.3 methoxy or
1.4 -CH=CH-COOR⁴ in which R⁴ is a hydrogen atom or (C₁-C₄)-alkyl,
and/or 2) monosubstituted in the a-position of the benzyl radical by
2.1 methyl,
2.2 hydroxymethyl,
2.3 carboxyl or
2.4 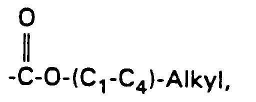
i) -(CH₂)ₙ-COOR⁵ in which
n is an integer from 1 to 8 and
R⁵ is
a) a hydrogen atom or
b) (C₁-C₄)-alkyl,
k) -CH₂-CH=CH-COOR⁶ in which R⁶ is (C₁-C₄)-alkyl,
l) pyridylmethyl,
m) 1-benzylimidazol-2-ylmethyl,
n) -CH(COOR⁶)₂, in which R⁶ is (C₁-C₄)-alkyl,
o) ω-morpholin-4-yl-(C₂-C₄)-alkyl,
p) 2-methylsulfinylethyl or
q) thienylmethyl,
with the exception of the compound of the formula I in which R¹ and R² are hydrogen atoms.

4. A compound of the formula I as claimed in claim 3 in which
R¹ is
a) a hydrogen atom,
b) (C₁-C₄)-alkyl,
c) 2-hydroxyethyl,
d) benzyl or
e) 2-methylallyl,
R² is
a) a hydrogen atom,
b) (C₁-C₆)-alkyl or
c) benzyl and
R³ is
a) a hydrogen atom,
b) (C₁-C₈)-alkyl,
c) cyclohexylmethyl,
d) ω-hydroxy-(C₂-C₈)-alkyl,
e) (ω-1)-(C₃-C₅)-alkenyl,
f) (ω-1)-(C₃-C₅)-alkenyl substituted on the (ω-1) carbon by methyl,
g) benzyl,
h) benzyl 1) monosubstituted or polysubstituted on the ring by
1.1 a halogen atom such as a fluorine or chlorine atom,
1.2 nitrile,
1.3 methoxy or
1.4 -CH=CH-COOR⁴ in which R⁴ is methyl or ethyl,
and/or 2) monosubstituted in the α-position of the benzyl radical by
2.1 methyl,
2.2 hydroxymethyl,
2.3 carboxyl,
2.4 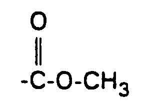 or
2.5 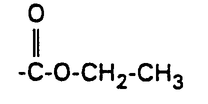
i) -(CH₂)ₙ-COOR⁵ in which
n is an integer from 1 to 8 and
R⁵ is
a) a hydrogen atom or
b) (C₁-C₂)-alkyl,
k) -CH₂-CH=CH-COOR⁶ in which R⁶ is (C₁-C₂)-alkyl,
l) 2-pyridylmethyl,
m) 1-benzylimidazol-2-ylmethyl,
n) -CH(COOR⁶)₂ in which R⁶ is (C₁-C₂)-alkyl,
o) ω-morpholin-4-ylethyl,
p) 2-methylsulfinylethyl or
q) 2-thienylmethyl,
with the exception of the compound of the formula I in which R¹ and R² are hydrogen atoms.

5. A compound of the formula I as claimed in claim 3 or 4 in which
R¹ is
a) a hydrogen atom,
b) methyl or
c) ethyl,
R² is
a) a hydrogen atom,
b) (C₁-C₄)-alkyl or
c) benzyl and
R³ is
a) a hydrogen atom,
b) (C₁-C₈)-alkyl,
c) cyclohexylmethyl,
d) ω-hydroxy-(C₂-C₄)-alkyl,
e) (ω-1)-(C₃-C₅)-alkenyl,
f) (ω-1)-(C₃-C₅)-alkenyl substituted on the (ω-1) carbon by methyl,
g) benzyl,
h) benzyl 1) monosubstituted or polysubstituted on the ring by
1.1 a fluorine or chlorine atom,
1.2 nitrile,
1.3 methoxy or
1.4 -CH=CH-COOR⁴ on the benzene ring in which R⁴ is methyl or ethyl,
and/or 2) monosubstituted in the a-position of the benzyl radical by
2.1 carboxyl,
2.2 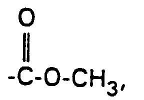 or
2.3 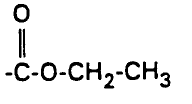 or
i) 2-thienylmethyl,
with the exception of the compound of the formula I in which R¹ and R² are hydrogen atoms.

6. A process for the preparation of the compound of the formula I as claimed in one or more of claims 3 to 5, which comprises
A) the reaction of a compound of the formula II: 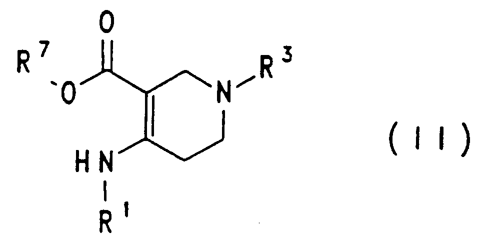 in which
R¹ is
a) a hydrogen atom or
b) (C₁-C₄)-alkyl,
R³ is
a) (C₁-C₈)-alkyl,
b) cyclohexylmethyl,
c) benzyl monosubstituted or polysubstituted on the ring by a halogen atom or methoxy as in formula I as claimed in claim 1 or
d) benzyl and
R⁷ is a lower alkyl radical, preferably methyl or ethyl,
with a compound of the formula III
R² - N = C = Y (III)
in which
R² is
a) (C₁-C₆)-alkyl,
b) benzyl monosubstituted or polysubstituted on the ring by a halogen atom or methoxy as in formula I as claimed in claim 1 or
c) benzyl and
Y is an oxygen atom,
to give a compound of the formula I 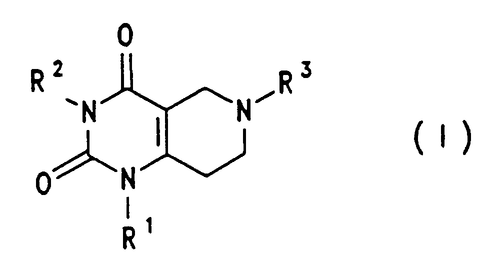 in which R² is as defined in formula III and R¹ and R³ are as defined in formula II, or
B) the reaction of a compound of the formula II in which R¹, R³ and R⁷ are as defined in formula II, with a compound of the formula III in which
R² is
a) (C₁-C₆)-alkyl,
b) benzyl monosubstituted or polysubstituted on the ring by halogen or methoxy as in formula I as claimed in claim 1, or
c) benzyl and
Y is a sulfur atom,
to give a compound of the formula IV 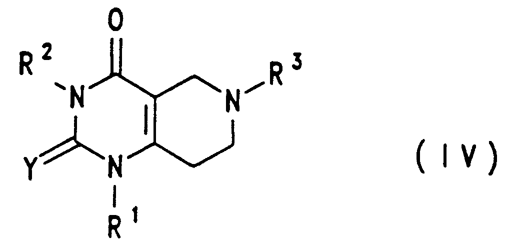 in which Y is a sulfur atom, R² is as defined in formula III and R¹ and R³ are as defined in formula II,
and the oxidation of the compound of the formula IV to give a compound of the formula I in which R² is as defined in formula III and R¹ and R³ are as defined in formula II, or
C) the reaction of a compound of the formula I in which R¹ is a hydrogen atom, (C₁-C₄)-alkyl or ω-hydroxy-(C₂-C₄)-alkyl, R² is a hydrogen atom, (C₁-C₆)-alkyl, benzyl or benzyl monosubstituted or polysubstituted on the ring by methoxy and R³ is a benzyl radical, to give a compound of the formula I in which R³ is a hydrogen atom and R¹ and R² are as defined, or
D) the reaction of a compound of the formula I and/or salts of the compound of the formula I in which R¹ and/or R³ are hydrogen atoms and R² is as defined in formula I, except for the hydrogen atom, with a compound of the formula VII
R⁸ - X (VII)
in which
X is
a) a halogen atom such as a chlorine, iodine or bromine atom,
b) a sulfonic acid ester radical or
c) a phosphoric acid ester radical and
R⁸ is as defined for R¹ and R³ in formula I, except for the hydrogen atom, those radicals R⁸ which carry an alcoholic hydroxyl group or a carboxyl group optionally being in protected form, and, after cleavage of the protecting group, conversion to a compound of the formula I, or
E) the reaction of a compound of the formula I in which R¹ and R² are as defined in formula I, except for (ω-1)-(C₃-C₅)-alkenyl with a hydrogen atom or methyl on the (ω-1) carbon atom, or benzyl monosubstituted or polysubstituted on the ring by nitrile or a bromine or iodine atom, and R³ is 2-methyl-sulfanylethyl, with an oxidizing agent, preferably a peracid, to give a compound of the formula I in which R¹ and R² are as defined and R³ is 2-methylsulfinylethyl, or
F) the reaction of a compound of the formula I in which R¹ and R² are hydrogen atoms, alkyl or benzyl according to formula I, and R³ is a hydrogen atom with a compound of the formula V 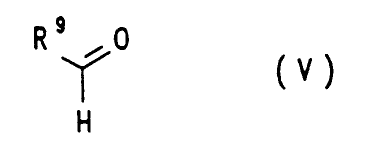 in which
R⁹ is
a) a hydrogen atom,
b) (C₁-C₇)-alkyl,
c) cyclohexyl or
d) phenyl,
under reductive reaction conditions to give a compound of the formula I.

7. A pharmaceutical containing an effective amount of at least one compound of the formula I as claimed in one or more of claims 3 to 5, or as obtained in claim 6, and/or at least one physiologically acceptable salt of a compound of the formula I, in addition to physiologically acceptable adjuncts and excipients and optionally other additives and/or other active ingredients.

8. A pharmaceutical as claimed in claim 7 for the prophylaxis and therapy of circulatory disorders and/or neurodegenerative diseases.

9. The use of at least one compound of the formula I as claimed in one or more of claims 3 to 5 for the preparation of pharmaceuticals for the prophylaxis and therapy of circulatory disorders and/or neurodegenerative diseases.

10. A process for the preparation of a pharmaceutical as claimed in claim 7 or 8, which comprises converting at least one compound of the formula I and/or at least one physiologically acceptable salt of such a compound and/or a compound obtained by the process as claimed in claim 6 to a suitable form of administration with physiologically acceptable adjuncts and excipients and optionally other additives and/or other active ingredients.

## Revendications

1. Utilisation d'au moins un composé de formule I 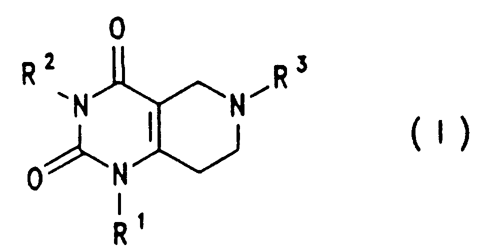 et/ou d'un sel physiologiquement acceptable du composé de formule I et/ou d'une forme éventuellement stéreoisomère du composé de formule I, dans laquelle
R¹ représente
a) un atome d'hydrogène,
b) un groupe alkyle en C₁-C₄,
c) un groupe ω-hydroxyalkyle en C₂-C₄,
d) le groupe benzyle,
e) un groupe benzyle une ou plusieurs fois substitué sur le noyau par
1) un atome d'halogène, tel qu'un atome de fluor, chlore, brome ou iode,
2) le groupe nitrile ou
3) le groupe méthoxy,
f) un groupe (ω-1)-alcenyle en C₃-C₅ ou
g) un groupe (ω-1)-alcényle en C₃-C₅ substitué sur l'atome de carbone (ω-1) par le groupe méthyle,
R² représente
a) un atome d'hydrogène,
b) un groupe alkyle en C₁-C₆,
c) le groupe benzyle,
e) un groupe benzyle une ou plusieurs fois substitué sur le noyau par
1) un atome d'halogène, tel qu'un atome de fluor, chlore, brome ou iode,
2) le groupe nitrile ou
3) le groupe méthoxy et
R³ représente
a) un atome d'hydrogène,
b) un groupe alkyle en C₁-C₈,
c) le groupe cyclohexylméthyle,
d) un groupe ω-hydroxyalkyle en C₂-C₈,
e) un groupe (ω-1)-alcenyle en C₃-C₅,
f) un groupe (ω-1)-alcényle en C₃-C₅ substitué sur l'atome de carbone (ω-1) par le groupe méthyle,
g) le groupe benzyle,
h) un groupe benzyle 1) une ou plusieurs fois substitué sur le noyau par
1.1 un atome d'halogène, tel qu'un atome de fluor, chlore, brome ou iode,
1.2 le groupe nitrile,
1.3 le groupe méthoxy ou
1.4 un groupe -CH=CH-COOR⁴, dans lequel R⁴ représente un atome d'hydrogène ou un groupe alkyle en C₁-C₄,
et/ou 2) substitué une fois en position α du radical benzyle par
2.1 le groupe méthyle,
2.2 le groupe hydroxyméthyle,
2.3 le groupe carboxyle ou
2.4 un groupe 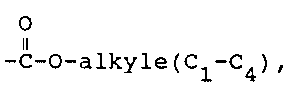
i) un groupe -(CH₂)ₙ-COOR⁵, dans lequel
n représente un nombre entier allant de 1 à 8, et
R⁵ représente
a) un atome d'hydrogène ou
b) un groupe alkyle en C₁-C₄,
k) un groupe -CH₂-CH=CH-COOR⁶, dans lequel R⁶ représente un groupe alkyle en C₁-C₄,
l) le groupe pyridylméthyle
m) le groupe 1-benzylimidazole-2-yl-méthyle,
n) un groupe -CH(COOR⁶)₂, dans lequel R⁶ représente un groupe alkyle en C₁-C₄,
o) un groupe ω-morpholino-4-yl-alkyle(C₂-C₄),
p) le groupe 2-méthylsulfinyléthyle ou
q) le groupe thiénylméthyle (thényle),
pour la fabrication de médicaments destinés à la prophylaxie et au traitement de troubles circulatoires et/ou de maladies neurodégénératives.

2. Utilisation selon la revendication 1, caractérisé en ce que l'on utilise au moins un composé de formule I dans lequel
R¹ représente
a) un atome d'hydrogène,
b) un groupe alkyle en C₁-C₄,
c) un groupe ω-hydroxyalkyle en C₂-C₄
d) le groupe benzyle,
e) un groupe benzyle une ou plusieurs fois substitué sur le noyau par
1) un atome de fluor ou de chlore,
2) le groupe nitrile ou
3) le groupe méthoxy,
f) un groupe (ω-1)-alcenyle en C₃-C₅ ou
g) un groupe (ω-1)-alcényle en C₃-C₅ substitué sur l'atome de carbone (ω-1) par le groupe méthyle,
R² représente
a) un atome d'hydrogène,
b) un groupe alkyle en C₁-C₆,
c) le groupe benzyle,
d) un groupe benzyle une ou plusieurs fois substitué sur le noyau par
1) un atome de fluor ou de chlore,
2) le groupe nitrile ou
3) le groupe méthoxy et
R³ représente
a) un atome d'hydrogène,
b) un groupe alkyle en C₁-C₈,
c) le groupe cyclohexylméthyle,
d) un groupe ω-hydroxyalkyle en C₂-C₈,
e) un groupe (ω-1)-alcenyle en C₃-C₅,
f) un groupe (ω-1)-alcényle en C₃-C₅ substitué sur l'atome de carbone (ω-1) par le groupe méthyle,
g) le groupe benzyle,
h) un groupe benzyle 1) une ou plusieurs fois substitué sur le noyau par
1.1 un atome de fluor ou de chlore,
1.2 le groupe nitrile,
1.3 le groupe méthoxy ou
1.4 un groupe -CH=CH-COOR⁴, dans lequel R⁴ représente un atome d'hydrogène ou un groupe alkyle en C₁-C₄,
et/ou 2) substitué une fois en position α du radical benzyle par
2.1 le groupe méthyle,
2.2 le groupe hydroxyméthyle,
2.3 le groupe carboxyle ou
2.4 le groupe 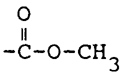 ou
2.5 le groupe 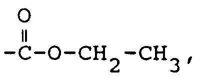
i) un groupe -(CH₂)ₙ-COOR⁵, dans lequel
n représente un nombre entier allant de 1 à 8, et
R⁵ représente
a) un atome d'hydrogène ou
b) un groupe alkyle en C₁-C₄,
k) un groupe -CH₂-CH=CH-COOR⁶, dans lequel R⁶ représente un groupe alkyle en C₁-C₄,
l) le groupe 2-pyridylméthyle,
m) le groupe 1-benzylimidazole-2-yl-méthyle,
n) un groupe -CH(COOR⁶)₂, dans lequel R⁶ représente un groupe alkyle en C₁-C₄,
o) un groupe ω-morpholino-4-yl-alkyle(C₂-C₄),
p) le groupe 2-méthylsulfinyléthyle ou
q) le groupe 2-thiénylméthyle.

3. Composé de formule I 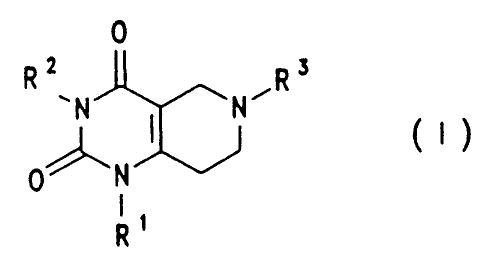 et sels physiologiquement acceptables du composé de formule I et formes éventuellement stéréoisomères du composé de formule I, dans laquelle
R¹ représente
a) un atome d'hydrogène,
b) un groupe alkyle en C₁-C₄,
c) un groupe ω-hydroxyalkyle en C₂-C₄,
d) le groupe benzyle,
e) un groupe benzyle une ou plusieurs fois substitué sur le noyau par
1) un atome d'halogène, tel qu'un atome de fluor, chlore, brome ou iode,
2) le groupe nitrile ou
3) le groupe méthoxy,
f) un groupe (ω-1)-alcenyle en C₃-C₅ ou
g) un groupe (ω-1)-alcényle en C₃-C₅ substitué sur l'atome de carbone (ω-1) par le groupe méthyle,
R² représente
a) un atome d'hydrogène,
b) un groupe alkyle en C₁-C₆,
c) le groupe benzyle,
d) un groupe benzyle une ou plusieurs fois substitué sur le noyau par
1) un atome de fluor, chlore, brome ou iode,
2) le groupe nitrile ou
3) le groupe méthoxy et
R³ représente
a) un atome d'hydrogène,
b) un groupe alkyle en C₁-C₈,
c) le groupe cyclohexylméthyle,
d) un groupe ω-hydroxyalkyle en C₂-C₈,
e) un groupe (ω-1)-alcenyle en C₃-C₅,
f) un groupe (ω-1)-alcényle en C₃-C₅ substitué sur l'atome de carbone (ω-1) par le groupe méthyle,
g) le groupe benzyle,
h) un groupe benzyle 1) une ou plusieurs fois substitué sur le noyau par
1.1 un atome de fluor ou de chlore,
1.2 le groupe nitrile,
1.3 le groupe méthoxy ou
1.4 un groupe -CH=CH-COOR⁴, dans lequel R⁴ représente un atome d'hydrogène ou un groupe alkyle en C₁-C₄,
et/ou 2) substitué une fois en position α du radical benzyle par
2.1 le groupe méthyle,
2.2 le groupe hydroxyméthyle,
2.3 le groupe carboxyle ou
2.4 un groupe 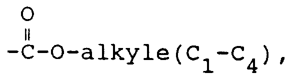
i) un groupe -(CH₂)ₙ-COOR⁵, dans lequel
n représente un nombre entier allant de 1 à 8, et
R⁵ représente
a) un atome d'hydrogène ou
b) un groupe alkyle en C₁-C₄,
k) un groupe -CH₂-CH=CH-COOR⁶, dans lequel R⁶ représente un groupe alkyle en C₁-C₄,
l) le groupe pyridylméthyle
m) le groupe 1-benzylimidazole-2-yl-méthyle,
n) un groupe -CH(COOR⁶)₂, dans lequel R⁶ représente un groupe alkyle en C₁-C₄,
o) un groupe ω-morpholino-4-yl-alkyle(C₂-C₄),
p) le groupe 2-méthylsulfinyléthyle ou
q) le groupe thiénylméthyle,
à l'exclusion du composé de formule I dans lequel R¹ et R² sont des atomes d'hydrogène.

4. Composé de formule I selon la revendication 3, dans lequel
R¹ représente
a) un atome d'hydrogène,
b) un groupe alkyle en C₁-C₄,
c) le groupe 2-hydroxyéthyle,
d) le groupe benzyle ou
e) le groupe 2-méthylallyle,
R² représente
a) un atome d'hydrogène,
b) un groupe alkyle en C₁-C₆ ou
c) le groupe benzyle et
R³ représente
a) un atome d'hydrogène,
b) un groupe alkyle en C₁-C₈,
c) le groupe cyclohexylméthyle,
d) un groupe ω-hydroxyalkyle en C₂-C₈,
e) un groupe (ω-1)-alcenyle en C₃-C₅,
f) un groupe (ω-1)-alcényle en C₃-C₅ substitué sur l'atome de carbone (ω-1) par le groupe méthyle,
g) le groupe benzyle,
h) un groupe benzyle 1) une ou plusieurs fois substitué sur le noyau par
1.1 un atome d'halogène, tel qu'un atome de fluor ou de chlore,
1.2 le groupe nitrile,
1.3 le groupe méthoxy,
1.4 un groupe -CH=CH-COOR⁴, dans lequel R⁴ représente le groupe méthyle ou éthyle,
et/ou 2) substitué une fois en position α du radical benzyle par
2.1 le groupe méthyle,
2.2 le groupe hydroxyméthyle,
2.3 le groupe carboxyle ou
2.4 le groupe 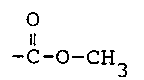 ou
2.5 le groupe 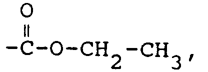
i) un groupe -(CH₂)ₙ-COOR⁵, dans lequel
n représente un nombre entier allant de 1 à 8 et
R⁵ représente
a) un atome d'hydrogène ou
b) un groupe alkyle en C₁-C₂,
k) un groupe -CH₂-CH=CH-COOR⁶, dans lequel R⁶ représente un groupe alkyle en C₁-C₂,
l) le groupe 2-pyridylméthyle
m) le groupe 1-benzylimidazole-2-yl-méthyle,
n) un groupe -CH(COOR⁶)₂, dans lequel R⁶ représente un groupe alkyle en C₁-C₂,
o) le groupe ω-morpholino-4-yléthyle,
p) le groupe 2-méthylsulfinyléthyle ou
q) le groupe 2-thiénylméthyle,
à l'exclusion du composé de formule I dans lequel R¹ et R² sont des atomes d'hydrogène.

5. Composé de formule I selon la revendication 3 ou 4, dans lequel
R¹ représente
a) un atome d'hydrogène,
b) le groupe méthyle ou,
c) le groupe éthyle,
R² représente
a) un atome d'hydrogène,
b) un groupe alkyle en C₁-C₄ ou
c) le groupe benzyle,
R³ représente
a) un atome d'hydrogène,
b) un groupe alkyle en C₁-C₈,
c) le groupe cyclohexylméthyle,
d) un groupe ω-hydroxyalkyle en C₂-C₄,
e) un groupe (ω-1)-alcenyle en C₃-C₅,
f) un groupe (ω-1)-alcényle en C₃-C₅ substitué sur l'atome de carbone (ω-1) par le groupe méthyle,
g) le groupe benzyle,
h) un groupe benzyle 1) une ou plusieurs fois substitué sur le noyau par
1.1 un atome de fluor ou de chlore,
1.2 le groupe nitrile,
1.3 le groupe méthoxy,
1.4 un groupe -CH=CH-COOR⁴, dans lequel R⁴ représente le groupe méthyle ou éthyle,
et/ou 2) substitué une fois en position α du radical benzyle par
2.1 le groupe carboxyle,
2.2 le groupe 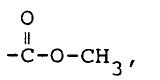
2.3 le groupe 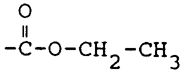 ou
i) le groupe 2-thiénylméthyle,
à l'exclusion du composé de formule I dans lequel R¹ et R² sont des atomes d'hydrogène.

6. Procédé pour la préparation du composé de formule I selon une ou plusieurs des revendications 3 à 5, caractérisé en ce que
A) on fait réagir un composé de formule II 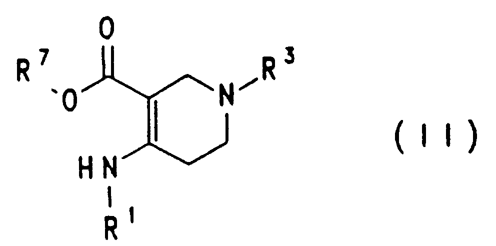 dans laquelle
R¹ est
a) un atome d'hydrogène ou
b) un groupe alkyle en C₁-C₄,
R³ est
a) un groupe alkyle en C₁-C₈,
b) le groupe cyclohexylméthyle,
c) un groupe benzyle une ou plusieurs fois substitué sur le noyau par un ou des atomes d'halogène ou par le groupe méthoxy, comme dans la formule I selon la revendication 1, ou
d) le groupe benzyle, et
R⁷ représente un radical alkyle inférieur, de préférence le groupe méthyle ou éthyle,
avec un composé de formule III
R²-N=C=Y (III)
dans laquelle
R² est
a) un groupe alkyle en C₁-C₆,
b) un groupe benzyle une ou plusieurs fois substitué sur le noyau par un ou des atomes d'halogène ou par le groupe méthoxy, comme dans la formule I selon la revendication 1, ou
c) le groupe benzyle et
Y représente un atome d'oxygène,
pour aboutir à un composé de formule I 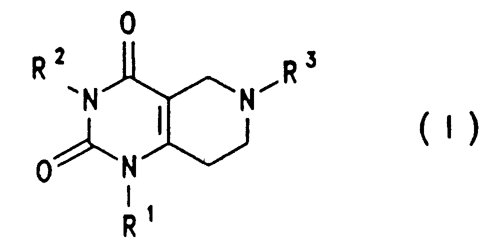 dans laquelle R² a la signification donnée dans la formule III et R¹ et R³ ont les significations données dans la formule II, ou
B) on fait réagir un composé de formule II, dans lequel R¹, R³ et R⁷ ont les significations données dans la formule II, avec un composé de formule III, dans lequel
R² est
a) un groupe alkyle en C₁-C₆,
b) un groupe benzyle une ou plusieurs fois substitué sur le noyau par un ou des atomes d'halogène ou par le groupe méthoxy, comme dans la formule I selon la revendication 1, ou
c) le groupe benzyle et
Y représente un atome de soufre,
pour aboutir à un composé de formule IV 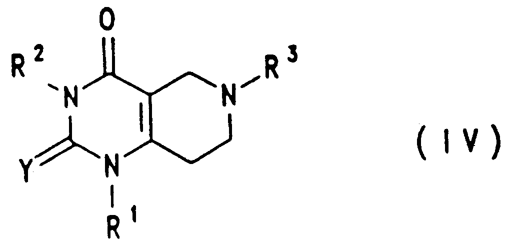 dans lequel Y représente un atome de soufre et R² a la signification donnée dans la formule III et R¹ et R³ ont les significations données dans la formule II, et on oxyde le composé de formule IV en un composé de formule I dans lequel R² a la signification donnée dans la formule III et R¹ et R³ ont les significations données dans la formule II, ou
C) on convertit un composé de formule I dans lequel R¹ représente un atome d'hydrogène ou un groupe alkyle en C₁-C₄ ou ω-hydroxyalkyle en C₂-C₄ et R² représente un atome d'hydrogène ou un groupe alkyle en C₁-C₆, benzyle ou un groupe benzyle une ou plusieurs fois substitué sur le noyau par le groupe méthoxy, et R³ représente le groupe benzyle, en un composé de formule I dans lequel R³ représente un atome d'hydrogène et R¹ et R² ont les significations indiquées, ou
D) on convertit en un composé de formule I un composé de formule I et/ou des sels du composé de formule I, dans lesquels R¹ et/ou R³ représentent un atome d'hydrogène et R² a la signification donnée dans la formule I, à l'exception de celle d'un atome d'hydrogène, avec un composé de formule VII
R⁸-X (VII)
dans laquelle
X représente
a) un atome d'halogène, tel qu'un atome de chlore, iode ou brome,
b) un reste d'ester d'acide sulfonique ou
c) un reste d'ester d'acide phosphorique et
R⁸ a la signification donnée pour R¹ et R³ dans la formule I, à l'exception de celle d'un atome d'hydrogène,
en mettant éventuellement sous une forme protégée les radicaux R⁸ qui portent un groupe hydroxyle alcoolique ou un groupe carboxyle, et après élimination du groupe protecteur, ou
E) on convertit un composé de formule I, dans lequel R¹ et R² ont les significations données dans la formule I, à l'exception de celles d'un groupe (ω-1)-alcényle en C₃-C₅ comportant un atome d'hydrogène ou le groupe méthyle sur l'atome de carbone (ω-1) ou du groupe benzyle une ou plusieurs fois substitué sur le noyau par le groupe nitrile ou l'atome de brome ou d'iode, et R³ est le groupe 2-méthylsulfinyléthyle, avec un oxydant, de préférence un peracide, en un composé de formule I dans lequel R¹ et R² ont les significations indiquées et R³ est le groupe 2-méthylsulfinyléthyle, ou
F) on fait réagir un composé de formule I dans lequel R¹ et R² représentent un atome d'hydrogène ou un groupe alkyle ou benzyle selon la formule I et R³ est un atome d'hydrogène, avec un composé de formule V 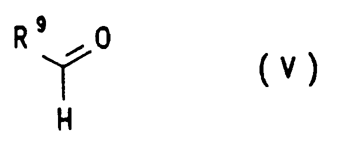 dans laquelle
R⁹ représente
a) un atome d'hydrogène,
b) un groupe alkyle en C₁-C₇,
c) le groupe cyclohexyle ou
d) le groupe phényle,
dans des conditions réactionnelles réductrices, pour aboutir à un composé de formule I.

7. Médicament, contenant une quantité efficace d'au moins un composé de formule I selon une ou plusieurs des revendications 3 à 5, ou tel qu'obtenu selon la revendication 6, et/ou d'au moins un sel physiologiquement acceptable d'un composé de formule I, en plus d'adjuvants et véhicules physiologiquement acceptables, éventuellement d'autres additifs et/ou d'autres substances actives.

8. Médicament selon la revendication 7, destiné à la prophylaxie et au traitement de troubles circulatoires et/ou de maladies néurodégénératives.

9. Utilisation d'au moins un composé de formule I selon une ou plusieurs des revendications 3 à 5, pour la fabrication de médicaments destinés à la prophylaxie et au traitement de troubles circulatoires et/ou de maladies neurodégénératives.

10. Procédé pour la fabrication d'un médicament selon la revendication 7 ou 8, caractérisé en ce que l'on met sous une forme d'administration appropriée au moins un composé de formule I et/ou au moins un sel physiologiquement acceptable d'un tel composé et/ou un composé obtenu conformément au procédé selon la revendication 6, avec des adjuvants et véhicules physiologiquement acceptables et éventuellement d'autres additifs et/ou d'autres substances actives.
